(19) Europäisches Patentamt / European Patent Office / Office européen des brevets

(11) **EP 4 145 133 A1**

(12) **EUROPEAN PATENT APPLICATION**

(43) Date of publication:
**08.03.2023 Bulletin 2023/10**

(21) Application number: **21306212.8**

(22) Date of filing: **03.09.2021**

(51) International Patent Classification (IPC):
**G01N 33/68** (2006.01)    **G16H 50/30** (2018.01)

(52) Cooperative Patent Classification (CPC):
**G01N 33/6893; G16H 50/20; G16H 50/30; G01N 2800/52**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Assistance Publique Hôpitaux de Paris**
  **75004 Paris (FR)**
- **Institut National de la Santé et de la Recherche Médicale (INSERM)**
  **75013 Paris (FR)**
- **ICM (Institut du Cerveau et de la Moelle Épinière)**
  **75013 Paris (FR)**
- **Centre national de la recherche scientifique**
  **75016 Paris (FR)**

- **Sorbonne Université**
  **75006 Paris (FR)**

(72) Inventors:
- **HANIN, Aurélie**
  **75013 PARIS (FR)**
- **NAVARRO, Vincent**
  **75013 PARIS (FR)**
- **CHAVEZ, Mario**
  **75013 PARIS (FR)**
- **DEMERET, Sophie**
  **75013 PARIS (FR)**

(74) Representative: **Flesselles, Bruno F.G.**
  **BF IP**
  **36 rue Jean de la Fontaine**
  **75016 Paris (FR)**

(54) **METHOD FOR PREDICTION OF MORTALITY, FUNCTIONAL OUTCOME AND RECOVERY AFTER STATUS EPILEPTICUS**

(57) The invention relates to the field of patient's care and describes method and systems, for prediction of mortality, functional outcome and recovery after status epilepticus, based on machine classifiers and logistic regression functions, and using biological markers and variables easily obtainable in intensive care units.

Figure 4

EP 4 145 133 A1

**Description**

The invention relates to the field of patient's care and describes method and systems, for prediction of mortality, functional outcome and recovery after status epilepticus, based on machine classifiers and logistic regression functions, and using biological markers and variables easily obtainable in intensive care units.

Status epilepticus (SE) is a life-threatening prolonged epileptic seizure.[1,2] The reported SE mortality ranges from 5% to 46%[2,3], and survivors frequently show impairment of their functional outcome at discharge, with inconsistent recovery after several months.[2,4]

The identification of valuable prognostic biomarkers is challenging due to the heterogeneity of SE etiology and clinical presentation. To help clinicians, various markers (demographic, clinical, biochemical, electrophysiological, or imaging) and four scales (STESS, EMSE, mSTESS, END-IT) have been proposed to predict SE outcome.[5-10] Except the END-IT, these scales mostly assess short-term mortality (death at discharge). Only STESS and mSTESS, built on pre-hospitalized clinical data, can be applied to all patients, as EMSE is available for specific etiologies and END-IT required MRI. Despite its key role for treatment decisions, the assessment of the functional outcome is, however, poorly studied.

Status epilepticus is associated with molecular and cellular changes that may induce brain injury and subsequent neurologic sequels.[11] Protein biomarkers (e.g. Neuron Specific Enolase, S100beta protein, progranulin) have been proposed to assess the brain injury.[12-18] We have also highlighted the role of lipid metabolism in SE excitotoxicity,[19-21] suggesting their usefulness as SE outcome biomarkers.

Machine learning (ML) models allow the integration of complex and heterogeneous data into personalized medicine systems. Although ML algorithms have been successfully used in the neurocritical care setting, they have never been applied to predict SE outcome.[22]

In this context, the present application shows that it is possible to use machine learning to identify demographic, clinical or biochemical markers that are relevant for the prediction of mortality at discharge, the functional outcome at discharge and recovery after 6-12 months. In particular, it is shown that relevant markers can be used either in machine learning algorithms or in other algorithms (such as regression, or Cox algorithms) to obtain functions or programs that can be used in *in vitro* methods for predicting mortality at discharge, the functional outcome at discharge and recovery after 6-12 months. The inventors assessed the prognosis value of a large number (67) of demographic, clinical or biochemical markers, and disclose a method that makes it possible to reduce the number of such markers so as to select the markers with the best relevance.

The quality of the tests was determined by drawing a Receiver Operating Characteristic (ROC) curve and measuring the Area Under Receiver Operating Characteristic curve (AUROC). The ROC curve is drawn by plotting the sensitivity versus (1-specificity), after classification of the patients, according to the result obtained for the diagnosis test, for different thresholds (from 0 to 1). It is usually acknowledged that the area under a ROC curve which has a value superior to 0.7 is a good predictive curve for diagnosis. The ROC curve has to be acknowledged as a curve allowing prediction of the quality of a diagnosis test. It is best for the AUROC to be as closed as 1 as possible, this value describing a test which is 100 % specific and sensitive.

It is reminded that

(1) sensitivity is the probability that the test provides a positive result for individuals having the condition sought (detection of true positives). The sensitivity is low when the number of false negatives is high. The sensitivity is calculated by the formula Se = (number of individuals having the condition in whom the test is positive)/(number of individuals having the condition in whom the test is positive + number of individuals suffering from the disease in whom the test is negative).

(2) specificity is the probability that the test is negative in the individuals not having the condition sought (non-detection of true negatives). The specificity is low when the number of false positives is high. The specificity is calculated by the formula Sp = (number of individuals not having the condition in whom the test is negative)/(number of individuals having the condition in whom the test is negative + number of individuals not having the condition in whom the test is positive).

(3) Positive predictive value (PPV): is the probability of having the condition if the test is positive (i.e. that the patient is not a false positive). The positive predictive value is calculated by the formula PPV = (number of individuals having the condition in whom the test is positive)/(number of individuals having the condition in whom the test is positive + number of individuals not having the condition in whom the test is positive).

(4) Negative predictive value (NPV): is the probability of not having the condition if the test is negative (that the patient is not a false negative). The negative predictive value is calculated by the formula NPV = (number of individuals not having the condition in whom the test is negative)/(number of individuals not having the condition in whom the test is negative + number of individuals having the condition in whom the test is negative).

Generally, a test for diagnosis or prognosis comprises

    i. a step of gathering information from the patient
    ii. a step of comparing said information with regards to thresholds
    iii. a step of deducing, from the difference between the patient's information and the threshold, whether the patient has a specific disease or the stage of the patient's disease.

As a matter of illustration

    i. the information that can be gathered from the patient can be gathered directly from the patient (such as images from NMR, scanner, radiography, contrast-enhanced computed tomography), or indirectly from the patient, such as from a biological sample that has been obtained from a patient (such as urine, blood sample..). The information can be presence (or absence) and/or level of specific biological markers, or elevated levels of patient's markers.
    ii. once the information is obtained, it is compared to different values / standards and the deviation with regards to these standards is assessed. As a matter of illustration, the level of some biomarkers shall be compared to the level usually observed in healthy patients and to the levels usually observed in patients with the disease. Thresholds may exist, where 95 % of patients having passed the threshold have the disease and 95 % of the patients not having passed the threshold do not have the disease. For diseases where multiple clinical stages can be determined, such thresholds can discriminate the different stages. In this step ii, one may compare various types of information to their respective standards, in order to be able to reach a diagnostic in step iii (as a matter of illustration, one can use the values and information obtained from measurement of various blood or plasma markers, images from scanner and of Body Mass Index).
    iii. the last step is actually making the diagnosis (or deciding of the prognosis) *i.e.* deciding whether or not the patient has the condition sought, taking, in particular, into account the information gathered from the patient, the thresholds as described above. The physician may also take into account other elements (such as the consistency of the information gathered or the like) to make the diagnostic.

The methods disclosed in the present application include a step (i.a)), which comprise the steps of modifying the information obtained from the patient in order to obtain a new type of information, which is the one that is compared to the standards in step ii. Such modification can the combination of the values of variables in a function and obtaining an end value. Alternatively, one can use a machine classifier to obtain an end value (which is actually a class for the patient (having or not having the condition), potentially with a probability. When multiple classes are used with a machine classifier, one shall preferably obtain, as the output, the probability, for each class, that the patient is in that class.

In a first aspect, the invention thus relates to a method for prognosis of the outcome of status epilepticus for a patient, comprising:

    a. Providing the values of at least three markers, including at least one biological marker,
    b. Combining the values provided in a) in order to obtain an end value

wherein the end value is indicative of the outcome of status epilepticus.

The method can also be used to predict the evolution of the patient that has status epilepticus.

This method is performed *in vitro* or *ex vivo.* In particular, this method is performed with the values measured or observed from patients and doesn't include obtaining these values. This method is preferably performed *via* a computer. As indicated above, the values may be normalized.

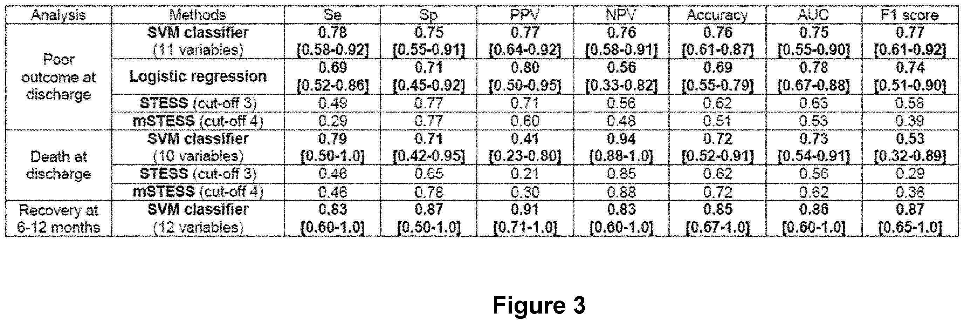

It is to be understood that the methods herein disclosed provide a prognosis on the evolution of the patient's clinical condition under all reasonable care, with the knowledge of the date of this application. Figure 3 shows the specificity, sensitivity, NPV and PPV of the various tests and methods described in details in the present specification. In particular, using the PPV and NPV of the tests, the physician will have an indication of the evolution of the patient's clinical condition, and can take any appropriate measure (discussion with family, preparation of follow-up and rehabilitation after release from the hospital and the like), depending on the result provided by the scores and methods. It is also to be understood that, due to the manner the scores and methods have been developed, the prognosis is not an absolute one (hence NPV and PPV are not at 1), but rather a relative prognosis, providing an information as to how the majority of the patients with the same result will evolve. Furthermore, the actual outputs (end result when obtained the regression scores, or the classification with the machine classifiers) can provide a estimation of the robustness of the prognosis (if the end result is far from the reference value when using the regression, or depending on the robustness (probability) calculated by the machine classifier). The methods herein disclosed are thus illustrative of bad prognosis, as there are not, currently, alternative or specific methods to treat the patients at risk.

As intended herein, a biological marker is a marker that is in biological media such as tissues, cells, or fluids.

In the preferred embodiment, the marker is measurable in the blood of the patient. For a biological marker, the value measured is the amount (or concentration) of the marker, potentially normalized.

Other markers can be used in the method, in particular clinical markers that relate to the clinical condition of the patient. For these markers, it is envisaged to assign them a discrete value (either binary 0/1, or not) depending on the patient's clinical condition at the time the marker is evaluated.

It is to be noted that the inventors herein disclose various tests using different markers, but also provides methods to select other values that could also be used.

In particular, the combination is performed in a processing device *via* a configured artificial machine learning classifier which generates, as the end value a class related to the evolution of the status of the patient, and potentially the probability that the patient is in this class (which can also be called label).

As an illustration of "a class related to the evolution of the status of the patient", if the evolution of the status of the patient is the death of the patient in intensive care unit (ICU), two classes can be designed: class 1: the patient will die during the stay in ICU, class 2: the patient will not die during the stay in ICU. The machine learning classifier can also indicate the probabilities (likelihood) (as an illustration 80% for being in class 1, 20% for being in class 2). Depending on the information / end value, output of the machine learning classifier, the physician will be able to adapt the treatment for the patient.

It is reminded that the principle of a machine learning classifier is to assign a given discrete output (a class) to input variables (here vectors consisting of the values of the markers used in the function). Various classifiers have been developed in the past years. One can cite artificial neural networks, k-nearest neighbors (KNN); clustering techniques, support vector machine, naive Bayes, random forest, decision tree, and the like.

In a preferred embodiment, the machine learning classifier is a support vector machine (SVM), preferably a two-classes support vector machine (i.e. that provides only two kinds of outputs (being in class 1 or in class 2). Preferably, it is a SVM with a Gaussian Kernel.

In another embodiment, the combination is performed through a mathematical function obtained multivariate analysis. Such function can be a binary logistic regression, a multiple linear regression or a time dependent regression. It is preferably a logistic regression function. Such function generates an end value that is compared to a reference value to predict the outcome of status epilepticus. It can be assimilated to a classifier (one type of outcome if the end value is above (or below) the reference value, and the other type of outcome if the end value is not above (or below) the reference value).

In another embodiment, the function is a Cox proportional hazard regression model adapted to predict an outcome at a given time (for instance recovery at 6 months).

The present examples disclose multiple markers that can be used in the context of the methods herein disclosed. In particular, Table 1 provides markers that have been studied by the inventors, and that are of particular interest for performing the methods herein described. It is, however, envisaged to use other markers (such as imaging or electrographic biomarkers). The markers that are listed in Table 1 have been selected as they can reflect the SE severity. Other markers could also have been included, such as inflammation markers (for instance CRP), lactates, or blood formula (in particular number of neutrophils, and/or of lymphocytes and/or ratio thereof). It is not necessary to use all markers of Table 1, as some are inter-correlated.

As indicated in the examples, the inventors were able to lower the number of markers that can be used (among the markers of Table 1), and to identify a set of markers that is of particular interest, as the results (methods and tests) obtained with these markers (subsets of this set, depending on the kind of outcome that is to be predicted) are of high quality (high AUROC, specificity, sensitivity, NPV and PPV) and as they are easy to be obtained from any patient that is admitted for status epilepticus.

Consequently, it is preferred when the at least one biological marker is selected from the group consisting of triglycerides (g/L), apolipoprotein B100 (g/L), apolipoprotein E (mg/dL), free cholesterol (g/L), ALAT (alanine aminotransferase) (UI/L), ASAT (aspartate aminotransferase) (UI/L), sodium (mM/L), potassium (mM/L), urea (mM/L), creatinine ($\mu$M/L), total cholesterol (g/L), HDL-cholesterol (g/L), esterified cholesterol (g/L), serum S100B protein (ng/mL), lipoprotein(a) (g/L), progranulin (ng/mL), chloride (mM/L), phospholipids (g/L), serum Neuron specific enolase (ng/mL) and gammaglutamyl transpeptidase (GGT) (UI/L).

In some embodiments, it is interesting to also use a "demographic" marker (the age of the patient). Consequently, the age of the patient can be combined with the values of the biological markers in order to obtain the end value.

As indicated in Table 1, one or more markers associated with the clinical condition of the patient (clinical marker) can also be used. As indicated above, such markers reflect the clinical condition of the patient (refractoriness or etiology of SE, functional state before the SE, previous history of epilepsy, duration of SE...). In some embodiments, at least one of such clinical marker is combined with the values of the biological markers (and optionally with the age of the patient) in order to obtain the end value.

After assessment of the clinical marker, one shall assign a value to it, in order to use such value in the methods herein disclosed. In some embodiments, the value associated with the clinical condition of the patient is selected from the group consisting of duration of status epilepticus (days), initial *modified Rankin score* (functional state of the patient before status epilepticus), and status refractoriness (1 is case of refractory status epilepticus, 0 in case of non-refractory status epilepticus). The modified Rankin score is well known in the art. It is used for measuring the degree of disability or dependence in the daily activities of people who have suffered a stroke or other causes of neurological disability. The scale runs from 0-6, running from perfect health without symptoms to death (Wilson et al, 2005, Stroke. 36 (4): 777-781)[41].

In a specific embodiment, the method is used to evaluate the risk of death of the patient in intensive care unit. In this embodiment, one can select markers from the group consisting of triglycerides (g/L), apolipoprotein B100 (g/L), apolipoprotein E (mg/dL), free cholesterol (g/L), ALAT (alanine aminotransferase) (UI/L), ASAT (aspartate aminotransferase) (UI/L), sodium (mM /L), potassium (mM /L), urea (mM /L), creatinine ($\mu$M/L). It is preferred when all of these markers are used.

In another specific embodiment, the method is used to assess the risk of poor outcome (i.e. death or worsening of clinical conditions) on discharge from the intensive care unit (mRSdischarge > mRSbaseline).

It is also to be noted that the methods herein disclosed can be repeated every day, which would then allow the physician to determine the evolution of the clinical condition of the patient. In this embodiment, one can select markers from the group consisting of total cholesterol (g/L), HDL-cholesterol (g/L), lipoprotein(a) (g/L), S100B highest serum value (ng/mL), progranulin (ng/mL), ASAT (UI/L), potassium (mM /L), chloride (mM /L), urea (mM/L), creatinine ($\mu$M/L), duration of status epilepticus before evaluation (days). It is preferred when all of these markers are used, in particular when the method is used by the way of a machine learning classifier. In this embodiment, one can also use status refractoriness (1 is case of refractory status epilepticus, 0 in case of non-refractory status epilepticus), free cholesterol (g/l) and phospholipids (g/l), as markers. This is particularly interesting when the method is to be performed *via* a logistic regression function.

Logistic regression gave similar results to SVM classifier (AUC=0.78 [0.67-0.88], positive predictive value, PPV=0.80, p<0.001; Figure 3) by using 3 variables: *"Refractory SE"*, a binary variable which takes the value of 1 in case of refractory SE or 0 in case of non-refractory SE, *"FC"* the concentration of free cholesterol (g/L) and *"phospholipids"* the concentration of phospholipids (g/L).

In particular, the function is
F1 = a1 + a2 * status refractoriness + a3 * free cholesterol + a4 * phospholipids, wherein

- -1 $\leq$ a1 $\leq$ -0.7, preferably -0.9 $\leq$ a1 $\leq$ -0.8
- 1.5 $\leq$ a2 $\leq$ 2, preferably 1.7 $\leq$ a2 $\leq$ 1.8
- 5 $\leq$ a3 $\leq$ 6, preferably 5.25 $\leq$ a3 $\leq$ 5.75
- -1.8 $\leq$ a4 $\leq$ -1.4, preferably -1.7 $\leq$ a4 $\leq$ -1.8.

In particular, F1 = -0.8741 + 1.7420 * status refractoriness + 5.5734 * free cholesterol - 1.6141 * phospholipids.

In another specific embodiment, the method is used to evaluate the degree of worsening expected at discharge from the intensive care unit (estimated accordingly to the modified Rankin scale). The approach is particularly relevant to better manage SE by providing information to physicians and families. In this embodiment, one can select markers from the group consisting of S100B highest serum value (ng/ml) during status epilepticus, initial modified Rankin score (functional state of the patient before status epilepticus) and creatinine ($\mu$M/l). It is preferred when all of these markers are used.

In particular, the function is

$$F1 = a1 + a2 * SB100max + a3 * modified\ Rankin\ initial + a4 * creatinine,$$

wherein

- 3 $\leq$ a1 $\leq$ 4, preferably 3.2 $\leq$ a1 $\leq$ 3.7
- 1.4 $\leq$ a2 $\leq$ 3, preferably 2.1 $\leq$ a2 $\leq$ 2.3
- -0.9 $\leq$ a3 $\leq$ -0.5, preferably -0.8 $\leq$ a3 $\leq$ -0.65
- -0.017 $\leq$ a4 $\leq$ -0.005, preferably -0.014 $\leq$ a4 $\leq$ -0.008

In particular, F2 = 3.5103 + 2.1758 * S100Bmax - 0.7390 * modified Rankin initial - 0.0117 * Creatinine

In another specific embodiment, the method is used to evaluate is the remote recovery from status epilepticus (i.e. recovery at 6-12 months: this corresponds to the recovery observed during a period extending from 6 to 12 months after discharge; consequently, the test allows to predict recovery at 12 months (if no other status epilepticus episode has occurred). Recovery is considered to be effective, if the mRS (modified Ranking score) at 6-12 months (mRSfollow-up) is below the mRS at discharge. The recovery may also be partial (mRSfollowup>mRSbaseline (initial mRS, before the status epilepticus episode; this mRS may be calculated a posteriori, using the clinical records and information available to the care team)) or total (mRSfollow-up=mRSbaseline). It is particularly relevant in the management of SE: a high probability of recovery at long-term may prompt clinicians to continue anesthesia for an extended period of time before deciding to discontinue life sustaining therapies. In addition, it is also relevant to provide accurate long-term prognostication to families. In this embodiment, one can select markers from the group consisting of age (years), apolipoprotein B100 (g/L), free cholesterol (g/L), phospholipids (g/L), maximal value of serum Neuron specific enolase (ng/mL), GGT (UI/L), sodium (mM/L), chloride (mM/L), urea (mM/L), creatinine ($\mu$M/L), duration of status epilepticus (days) and initial modified Rankin score. It is preferred when all of these markers are used.

In a specific and preferred embodiment, the method is computer implemented. In particular, the method comprises:

a. receiving, in a processing device, signal data representing values of at least three markers, including at least one biological marker, and optionally the age of the patient,
b. processing said signal data by the processing device, via a configured classifier, in order to generate an output indicative of the outcome of status epilepticus.

As indicated above, in some embodiments, the classifier can be a machine learning classifier (in particular a configured support vector machine) or a classifier that applies a mathematical formula to the data to provide an end result, and wherein the input data is assigned to a class if the end value is above (or below) a reference value, and to another class if the end value is not above (or below) the reference value.

In some embodiments, the data received by the processing device has been normalized. In particular, normalization is performed by

- calculating the arithmetic mean of the values of the markers
- calculating the variance of the values population
- multiplying the values by an adequate coefficient so as to maintain their mean constant and set of their variance equal to one (1),

thereby obtaining a set of normalized values.

It is preferred to perform such normalization when a support vector machine is used, as a machine classifier. Indeed, since the classes depend on the distance to the hyperplan, it is preferred to ensure that all variables have similar ranges of values, in order to give a weight to important to values with high values or ranges, and to avoid that prediction depends only on such variables with the highest scales.

The method of the invention can thus be considered as a method for prognosis of the outcome of status epilepticus for a patient, comprising:

a. providing values of at least three markers, including at least one biological marker, and optionally age of the patient;
b. calculating the mean of the values of a), and normalizing the values in order to set their variance equal to one, while maintaining their mean constant, thereby obtaining normalized values,
c. combining the normalized values obtained in b) in a configured algorithm, in particular by applying such values to a machine learning classifier (a support vector machine) configured to process the values,
d. obtaining an end result or an output, in particular assignment of the values of a) to a given class, wherein said end result or output is indicative of the outcome of status epilepticus.

As illustrations

- if the training of the machine learning classifier was performed with output two classes (depending on the outcome): the output will provide the class that is the most likely (and if appropriate, the likelihood of being in this class), the outcome of status epilepticus being thus associated with this class.
- if the training of the machine learning classifier performed with more than two classes (for instance good recovery at 6-12 months, average recovery at 6-12 months, bad recovery at 6-12 months), the output will provide the most likely class (and if appropriate, the likelihood of being in this class). The output may also include the likelihood for each class.

In a specific embodiment, the steps of normalizing the values and/or of processing the values (potentially normalized) and calculating the end result and/or obtaining the output are performed in a location that is remote from the patient's bed or from the one of step a) (inputting or providing the values). In practice, an operator enters the values in an electronic form, and the values are sent to a distant server, where the normalization and processing of the values is performed. Such sending is performed according to any method known in the art, such as by the internet or by a phone line. It is preferred when communication between the distant server and the device on which the electronic form is completed is encrypted. In practice, the operator may be an employee of a biological laboratory (in which the values of biological markers are measured), or by a hospital employee, in particular in case clinical data is also used.

Once the output or the end result is obtained, it is sent to (or made available to) the physician, by any method known in the art (such as by email, by text message, through a dedicated phone or computer application, directly to a hospital server... ).

In summary, it is envisaged that the values used in the methods herein disclosed are sent to a remote machine or server so as to obtain the end result/output and that the output is sent to a physician.

The methods and scores herein disclosed can be used to easily evaluate the impact of a new neuroprotective or antiepileptic therapeutic on the outcome and the evolution of the patient over patient. It can also be used to define a targeted, sufficiently homogenous, population for further clinical trials in order to permit precise estimation of treatment effect.

In particular, the methods and processes may be of particular advantage and interest in the process of development of a new drug or medicament, during clinical trials.

Using the outcome predicted for the patients included in the clinical trial, it is possible to determine whether a given substance (active ingredient) is able to lead to a better outcome (whether survival, improvement or no worsening at discharge...). Depending on the expected activity of the drug, it is also possible to select the patients that are the most susceptible to respond to the substance.

One can thus perform a method to determine whether a given substance of interest presents a positive action on patient with status epilepticus. Such method is part of the invention. This method would comprise the step of performing the method as disclosed above (combining the values of biochemical markers and potentially other variables in function and tests as herein disclosed) for various patients of a cohort. The study is performed on a cohort of patients. In fact, one should perform the study on a number of patient high enough to obtain statistically relevant results for the molecule that one desires to test (substance or drug of interest), and eliminate the inter-patients variability. The substance of interest will preferably be compared to a placebo, according to the best clinical practices.

The study is performed on a patient cohort, according to a protocol that could be as follows, for each patient:

- The predicted outcome is calculated for the patient before administration of the substance to be tested
- The actual outcome is observed after the patient has received the substance to be tested (which can thus be the substance of interest, or a placebo)

Any appropriate statistical analysis can be performed to evaluate whether there is a variation of the observed outcome as compared to the expected (predicted) outcome, and hence whether the substance of interest has an actual activity.

It is preferred when the cohort of patients (the number of patients on which the substance of interest will be tested) contains at least 10 patients, preferably at least 20 patients, or more preferably at least 50 patients. The person skilled in the art will determine the adequate number of patients in order to obtain results that are statistically significant.

It is possible to have multiple sub-cohorts, with patients of one sub-cohort receiving the substance of interest to be tested, patients of another sub-cohort receiving the placebo and patients of one (or more) sub-cohort(s) receiving the positive control.

As indicated above, using the methods herein disclosed, it is also possible to select sub-groups of patients that are the most susceptible to respond to the substance of interest. If the substance of interest is to be administered during the stay at ICU, to improve clinical condition at release, the tests predicting death or worsening are particularly appropriate. If the substance of interest is intended to improve recovery, the methods and tests pertaining to the clinical condition at 6-12 months are of great interest. such method thus provides an objectivization of the activity of the drug, as it can be used on patients for which evolution of the clinical status is known.

The invention also relates to a method for producing a machine learning classifier capable of prognosis of the outcome (evolution) of status epilepticus for a patient, comprising:

a. storing in an electronic database patient data comprising

i. input data consisting of values (preferably normalized) of at least three markers, including at least one biological marker, and optionally the age of the patient, and

ii. the outcome of status epilepticus observed for the patient at a date set after collection of the data (it is preferred when the outcome is organized in two classes (outcome 1 / outcome 2 such as recovery/no recovery, death/no death...));

b. providing a machine learning system; and

c. training the machine learning system using the patient data, such that the machine learning system is trained to assign the patient data to one of the classes, so that it can produce a prognosis of the outcome of status epilepticus for a patient when exposed to input data from the patient (the prognosis being associated with the class which is outputted by the machine learning system).

This method can be performed using patient data, used in a. above, obtained for 20 patients or more. It is preferred when the number of patients in a class is at least 5, more preferably at least 10, to be able to obtain a model that is sufficiently trained. It is preferred when the number of patients is essentially the same in the various groups in which the patients are classified (i.e., if two groups are envisaged, the number of patients shall be essentially the same, or the repartition of the patients is preferably about 40-60%, or about 45-55% between the two groups). However, in the context of the present application, the inventors have shown that it was possible to develop a method using SVM with a 20-80% repartition: indeed, the method disclosed for prediction of patient death was obtained with such a 20-80 patients repartition. To prevent any bias related to class-imbalanced training datasets, on can apply the Synthetic-Minority-Oversampling-TEchnique (SMOTE) that oversamples new points of the minority class, within each cross-validation, based on the similarities between available data[42].

The machine learning system (or machine learning classifier) is any system known in the art (neural network, clustering techniques, support vector machine, logistic regression, naive Bayes, random forest, decision tree...). Of particular interest are neural network systems, or support vector machine. The inventors have shown that using a support-vector machine classifier as the classification artificial system in the machine learning system made it possible to obtain very interesting results. In particular, the support vector machine is with a kernel (notably a Gaussian kernel). When the machine-learning classifier is a neural network, it is preferably a convolutional neural network. The training is supervised, as the output expected for the input data is indicated during training.

As an illustration, in the training phase, binary SVM models can be built by using training data (vectors with the values of considered markers) labelled or predefined into two set groups (as an illustration, are herein described the classes good/poor outcome, death/survival, recovery/non recovery as detailed above, and in the examples). The SVM algorithm will estimate the hyperplane which best separates and distinguishes data of the two classes (the "decision function").

SVM classifiers are of particular interest because of their robustness for modeling complex data, without any prior assumption about the underlying distribution.

In addition, since it is usually not possible, using this kind of vectors, to obtain a linear separation, the SVM shall use a transformation function (kernel) to project the data into a higher dimensional space; as known in the art, input data that cannot be linearly distinguished in the original space may become separable after transformation into the new high-dimensional feature space. Although linear or polynomial kernel functions could be envisaged, SVM models with a Gaussian kernel may often be best adapted as being more versatile and powerful than such linear or polynomial kernel functions. For this particular application, one can use a kernel width parameter $\gamma$ set to be the median pairwise distance among training points.

In the subsequent testing phase, the SVM model is used to predict the class to which a new patient belongs. Given training labelled data, the learned SVM model computes a decision, or scoring function, to predict the label of any new test input data (vector with the values of considered markers from a new, unseen patient). Therefore, for a given test patient, the prediction (SVM) model is built using data of all patients in the training phase.

Data of tested patient is presented in the same way as the data used for training, and constitute the input values of the learned model. For this tested patient, the output of the SVM classifier (a binary response) will be the outcome (or evolution) prediction of the status epilepticus.

Although the examples of the application describe SVM with a binary (two-classes) scenario, obtaining a multiclass classification is within the reach of one skilled in the art, and can be accomplished by using, for instance, an approach of one-versus-all classes.

Training of a neural network is performed similarly. Input data comprising the patient's values (whether normalized or not) and the label (output class) is provided as the training material to the neural network. One of skill in the art can determine the appropriate number of layers that should be used, in order to optimize the reliability of the output while minimizing the calculus time and resources.

The inventors have shown that it is possible to build various machine classifiers, making it possible to predict various outcomes for the status epilepticus patients.

Consequently, one can obtain a machine classifier, using the following classes and rules:

a. For the good/poor outcome classification.

i. "Good outcome" class when clinical conditions of patient on discharge are equal than initial clinical conditions

ii. "Poor outcome" class when clinical conditions of patient on discharge are worse than initial clinical conditions

b. For the death/survival classification:

i. "Death" class when patient dies
ii. "Survival" class when patient survives

c. For the Recovery/Non recovery classification:

i. "Recovery" class when clinical conditions of patient at long term are better than clinical conditions at discharge
ii. "Non recovery" class when clinical conditions of patient at long term are equal or worse than clinical conditions at discharge

As indicated, since the methods are used for the prognosis, the outcome is at a time that is later than the time on which the values of the markers are obtained.

It has been described above a method where the patient's markers' values are entered at one location and are processed at another remote location (and the outcome is then made available to a physician).

In another embodiment, computation of the marker's value is performed on the device that receives the values. It may be a computer, a smartphone or a dedicated device.

The invention thus also relates to a device comprising:

a. at least one interface for entering patient data comprising values of at least three markers, including at least one biological marker, and optionally age of the patient; and
b. a processing unit comprising at least one processor; and
c. at least one non-transitory computer-readable medium comprising program instructions that, when executed by the at least one processor, causes the device to:

i. process the patient data, if necessary to standardize the unities of the values;
ii. provide patient data, optionally standardized, to a machine learning classifier
iii. process patient data by the machine learning classifier, so as to obtain an output from the machine learning classifier, said output being a prognosis of the outcome [evolution] of status epilepticus for a patient when exposed to input data from the patient.

In another embodiment, the invention relates to a device comprising:

a. at least one interface for entering patient data comprising values of at least three markers, including at least one biological marker, and optionally age of the patient; and
b. a processing unit comprising at least one processor; and
c. at least one non-transitory computer-readable medium comprising program instructions that, when executed by the at least one processor, causes the device to:

i. process the patient data, if necessary to standardize the unities of the values;
ii. send patient data, optionally standardized, to a machine learning classifier.

Such device is particularly interesting when the classifier is located on a remote server.

As described in the examples, the inventors also provided a method to identify the most interesting markers that can be used in methods of the invention.

For the SVM analysis, the most "non-significant" variables were removed one by one by a pruning procedure:

(i) All markers considered to be introduced in the model are used to produce a first predictive model, the area under the receiver operating curve (AUC) being calculated for this model
(ii) each variable (marker) is removed and a new model is then produced without such removed variable, and the AUC is calculated.
(iii) the variable without which the model had the highest AUC is then definitively removed; and
(iv) the procedure was repeated with the remaining variables (removal of each variable, production of models, calculation of AUC and definitive removal of the value without which the AUC is the highest.

The procedure is then stopped when the AUC of the new models is lower than the AUC previously obtained with the model of the previous round. In the first rounds, the AUC may indeed increase when the variable that is to be definitely removed is has no particular impact on the model, so that it created background noise and lower the quality of the model (as determined by the AUC).

Using this procedure, it is thus possible to identify the set of the most important markers among the initial set of markers. This is interesting, as it reduces the computer resources for performing the methods and obtaining the results, and facilitates the possibility of obtaining all data of the patient upon admission in ICU (the lower the number of markers, the easier to obtain them). In particular, markers associated with the lipid metabolism or serum electrolytes are of particular interest.

In order to identify the most relevant markers when performing a regression analysis, a backward stepwise regression procedure was performed with a 1000-fold cross-validation procedure. At each fold, the most significant variables (X variables) were obtained. After 1000 folds, a percentage for each variable was obtained, representing the number of times the variable was selected to best predict the outcome. The three most frequently found variables were selected. As indicated in the examples, only three variables were selected as the number of patients for which the variables were available was not very important. Indeed, to avoid overfitting the inventors decided to limit the number of used variables. The inventors considered that it was not possible to use more than 1 variable for 10 patients. When comparing two groups of patients (the first group with 26 patients and second group with 34 patients), if the lowest group is composed by 26 people it is not possible to use more than 2,6 variables (=2 variables) to predict the group for a new patient. The tests herein specifically disclosed have been obtained using the retained variables accordingly to the variables most frequently found by the stepwise backward regression procedure. One immediately understands that other regression formulas can be obtained with a larger number of variables, if a higher number of patients is available. The most important element, here, is that the inventors showed that it is possible to use biological markers easily available at the patient's bedside and routinely measured in ICU to predict evolution of status epilepticus. These methods for selecting relevant markers are also part of the invention.

The invention also relates to methods for treating a patient with status epilepticus, comprising performing one of the prognosis method herein disclosed, and adapting the treatment of the patient, depending on the result of the method. In particular, when one predicts that the patient will show good recovery, it is possible to adapt the treatment by anticipating rehabilitation of the patient. If one predicts a poor outcome out of ICU, one could adjust the amount (increase or decrease) or the nature (change either the drug in the same class or change the class of the drug) of the drug provided to the patient. It is reminded that treatment includes use of benzodiazepines (including diazepam, lorazepam, midazolam, clonazepam), of phenytoin, of fosphenytoin, of phenobarbital, of valproate, of levetiracetam or of anesthetics (propofol, ketamine, midazolam, thiopental) in case of refractory SE. One can also adjust the amount of oxygen provided to the patient, control the glucose, metabolites, hyperthermia. Depending on the predicted outcome, adapting the therapeutic treatment can also include providing sedation to the patient or increasing the amount of sedative, or extending the sedation. The methods herein disclosed can thus be used by the physicians to adapt the treatment so as to be able to modify the predicted outcome (it is indeed reminded that the prediction is made at a specific time and can evolve overtime in particular if the treatment is adapted) The physician shall thus adapt, as it goes along, and on a case-by case basis, the treatment initially proposed and provided, depending on the predicted outcome for the patient.

**DESCRIPTION OF FIGURES**

Figure 1: Flow Chart of the study population.

Figure 2: Prognosis value of selected markers in predicting poor outcome and mortality at discharge. The variables in bold represent the variables significantly associated with the risk of poor outcome or mortality at discharge. *Markers not considered for multivariate analyses. Abbreviations: ALT = Alanine Aminotransferase; AST = Aspartate Aminotransferase; AU = Arbitrary Unit; ICU = Intensive Care Unit; mRS =modified Rankin Score; NSE = Neuron Specific Enolase; SE = Status Epilepticus

Figure 3: Predictive performance of the scores obtained by SVM classifier and logistic regression. The values are represented as mean [CI 95%]. Abbreviations: AUC = Area Under the receiver operating characteristic Curve; NPV = negative predictive value; PPV = positive predictive value; Se = sensitivity; Sp = specificity; SVM = Support Vector Machine F1 score is calculated as: 2*Se*PPV/(Se+PPV). The most relevant markers for poor outcome at discharge are: total cholesterol, HDL-cholesterol, lipoprotein(a), S100B highest serum value, progranulin, aspartate aminotransferase, potassium, chloride, urea, creatinine, SE duration before enrollment. The most relevant markers for death at discharge are: triglycerides, apolipoprotein B, apolipoprotein E, free cholesterol, alanine aminotransferase, aspartate aminotransferase, sodium, potassium, urea, creatinine. The most relevant markers for recovery at 6-12 months are: age, apolipoprotein B, free cholesterol, phospholipids, NSE highest serum value, gamma GT, sodium, chloride, urea, creatinine, total SE duration and mRS$_{baseline}$.

Figure 4: General scheme of the prediction method

A). Scheme of the cross-validation procedure. The ML classifiers used 70% of the observations to train the model; and then the remaining 30% of data were used to test the prediction performance. A first step of variable selection was performed for logistic regression: the 3 most frequently found variables were retained for the prediction performance. A cross-validation procedure was used with 1000 folds. B). SVM classifier prediction optimization. The SVM classifier prediction performances were secondary optimized by selecting the most relevant variables (var.). The "non-significant" variables were removed one by one by a pruning procedure: (i) The area under the receiver operating curve (AUC) values were obtained by cross-validation, after removal of each variable; (ii) the variable without which the model had the highest AUC was removed; and (iii) the procedure was repeated with the remaining variables.

## EXAMPLES

## Example 1. Summary and abbreviations

### Background and objectives

The identification of prognostic biomarkers that would apply to all status epilepticus (SE) patients is challenging due to clinical presentations heterogeneity. Here, we aimed to apply a data driven approach using machine learning (ML) models to identify predictive markers of mortality, functional outcome and recovery.

### Methods

SE patients admitted in the Pitié-Salpêtrière Hospital were enrolled between February 2013 and June 2020. Patients had a follow-up evaluation at 6-12 months after discharge. Their clinical outcome was assessed using the modified Rankin Scale. Sixty-seven (67) demographic, clinical and biochemical markers were selected so as to was evaluate their prognosis significance (Table 1). The biochemical markers were evaluated upon admission. ML models, obtained by support vector machine (SVM) and logistic regression models, were trained to predict mortality and functional outcome at discharge, and recovery at long-term. Their performances were compared to those of previous scales STESS and mSTESS.

### Results

Eighty-one patients were enrolled. Forty-six patients had a poor outcome at discharge (i.e. death or worsening of clinical conditions) while 35 patients had a good outcome (i.e. clinical steady state). Among the 46 patients, 14 died during the hospital stay, 14 had persistent disability at 6-12 months and 18 presented with a recovery at 6-12 months. ML models yielded predictions with the following area under the receiver operating characteristic curve (AUC) scores: 0.75 [0.55-0.90] (SVM) and 0.78 [0.67-0.88] (logistic regression) for poor outcome at discharge; 0.73 [0.54-0.91] (SVM) for mortality at discharge; and 0.86 [0.60-1.0] (SVM) for recovery at 6-12 months. Previous scales provided lower prediction for the poor outcome (AUC STESS=0.63; mSTESS= 0.53) and the mortality (AUC STESS=0.56; mSTESS=0.62).

### Conclusions

ML models significantly outperformed STESS and mSTESS scales in predicting outcome after SE. Furthermore, ML models allow the recovery prediction at long-term. They can be straightforwardly applied for all hospitalized SE patients. These tools might be used in clinical routine to monitor SE patients, to follow the impact of a new therapeutic, or to define a targeted and sufficiently homogenous population for further clinical trials in order to permit precise estimation of treatment effect.

### Abbreviations

AUC = Area Under the receiver operating characteristic Curve; CSF = CerebroSpinal Fluid; FC = Free Cholesterol; ICU = Intensive Care Unit; ML = Machine Learning; mRS = modified Rankin Score; NORSE = New-Onset Refractory Status Epilepticus; NPV = Negative Predictive Value; PPV = Positive Predictive Value; RSE = Refractory Status Epilepticus; S100B = S100-beta protein; SE = Status Epilepticus; SVM = Support Vector Machine

**Example 2. Methods**

*Study design, setting and participants*

Eighty-one (81) consecutive adult patients admitted with SE in Pitié-Salpêtrière Hospital between February 2013 and June 2020 were included in the study. Patients with post-anoxic SE were excluded, as they required significantly different management and had the worst outcomes.

This study received approval from the University Ethic Committee (2012, CPP Paris VI). All patients or relatives were informed and provided their consent. The study design and report are in accordance with the Strengthening the Reporting of Observational Studies in Epidemiology reporting guidelines.[23]

*Variables selection and data extraction*

The prognosis significance of 67 features (see details in Table1),[5,6,8,13,14,21,24,25] was studied, including: demographic (age), clinical (previous history of epilepsy, SE etiology, SE refractoriness, SE duration [i.e. the SE end was defined as the absence of seizures after the anesthetics withdrawal], consciousness at enrollment) and biochemical markers including routine laboratory blood measures, brain injury biomarkers, routine lipid biomarkers, precursors and metabolites of cholesterol. The clinical data and routine laboratory measures were extracted from medical records.

**Table 1: Demographic, clinical and biochemical markers**

| **Demographic marker (1)** | Age |
|---|---|
| **Clinical markers (12)** | |
| *SE subtype (3)* | Refractory SE, Super-refractory SE, Prolonged-super-refractory SE |
| *SE etiology (4)* | Acute, Remote, Progressive, Unknown |
| *Others clinical markers (5)* | Previous history of epilepsy, SE duration, mRS baseline, GCS score at enrollment, FOUR score at enrollment |
| **Biochemical markers (54)** | |
| *Routine laboratory blood measures (9)* | Sodium, Potassium, Chloride, Urea, Creatinine, aspartate aminotransferase, alanine aminotransferase, gamma GT, lactates |
| *Brain injury biomarkers in blood (5)* | Neuron Specific Enolase, S100-beta protein, progranulin *(if multiple recordings of the measure were available, we recorded the first one and the maximum value)* |
| *Brain injury biomarkers in CSF (3)* | Neuron Specific Enolase, S100-beta protein, progranulin |
| *Routine blood lipid biomarkers (17)* | Total cholesterol (TC), triglycerides, HDL-cholesterol (HDL-C), LDL-cholesterol (LDL-C), TC/HDL-C, apolipoprotein A1 (ApoA1), apolipoprotein B (ApoB), ApoA1/HDL-C, ApoB/LDL-C, lipoprotein(a), apolipoprotein E, lipoprotein-associated phospholipase A2, free cholesterol, esterified cholesterol (EC), cholesterol esterification ratio (EC/TC), phospholipids (PL), TC/PL |
| *Routine CSF lipid biomarkers (1)* | Apolipoprotein E |
| *Precursors and metabolites of cholesterol in blood (10)* | 27-hydroxycholesterol, 25-hydroxycholesterol, 24-hydroxycholesterol, cholesterol, sitosterol, dihydrolanosterol, lanosterol, desmosterol, cholestanetriol, 7-ketocholesterol |
| *Precursors and metabolites of cholesterol in CSF (9)* | 27-hydroxycholesterol, 25-hydroxycholesterol, 24-hydroxycholesterol, cholesterol, sitosterol, dihydrolanosterol, lanosterol, desmosterol, cholestanetriol |
| Abbreviations: CSF = Cerebrospinal fluid; FOUR score = Full Outline of UnResponsiveness score; GCS = Glasgow Coma Scale; ICU = Intensive Care Unit; mRS = modified Rankin Score; SE = Status Epilepticus | |

The study presented in these examples was performed using these 67 markers. One could also add or use other markers, such as inflammation markers, lactates, blood composition (neutrophils, lymphocytes...).

*Biochemical analyses*

The biochemical markers were assessed upon admission in intensive care unit (ICU). Neuron Specific Enolase (NSE) and S100beta protein (S100B) assays were performed using immunofluorimetric assays and electrochemiluminometric sandwich immunoassays (Kryptor® and Modular®E170, Roche Diagnostics), respectively. Progranulin measurements were obtained, in duplicated, using the progranulin-human-ELISA kit (Adipogen).

Total cholesterol (TC), triglycerides, HDL-cholesterol were measured by enzymatic methods; and apolipoprotein A1 and apolipoprotein B100 by immunoturbidimetric method on Cobas analyzer (Roche). Phospholipids and free cholesterol (FC) were analyzed by colorimetric method on Konelab analyzer (Thermo Fisher Scientific). Esterified cholesterol (EC) was calculated by difference (EC=TC-FC). Lipoprotein(a) and apolipoprotein E were measured by immunonephelemetric method on BNII analyzer (Siemens).

An ultra-performance liquid chromatography-tandem mass spectrometer (UPLC-MS/MS) with isotopic dilution method was used to measure sterols (cholesterol, lanosterol, dihydrolanosterol, desmosterol, sitosterol, cholestanetriol) and metabolites of cholesterol (24-hydroxycholesterol, 25-hydroxycholesterol, 27-hydroxycholesterol, 7-ketocholesterol), both in blood and in cerebrospinal fluid (CSF).[21]

*Outcome assessment*

The global outcome was assessed from medical records, or by in-person or a telephone structured interview at discharge (called *discharge)* and at 6-12 months (called *follow-up)* using the 7-point version of the modified Rankin Scale (mRS), rated from death (6) to symptom-free full recovery (0).[26] The same scale was used to assess the functional state before SE (called *baseline).* If a patient had several follow-up evaluations, we considered the last evaluation as the $mRS_{follow-up}$. If a patient had a good outcome at discharge ($mRS_{discharge}=mRS_{baseline}$) and was not followed at 6-12 months, we considered the $mRS_{discharge}$ for the follow-up evaluation.

Four analyses were performed: (i) prediction of poor outcome at discharge (i.e. mortality or worsening of clinical conditions; $mRS_{discharge}>mRS_{baseline}$); (ii) prediction of the degree of worsening at discharge (i.e. $1<mRS_{discharge}-mRS_{baseline}<6$); (iii) mortality prediction at discharge (i.e. $mRS_{discharge}=6$); and (iv) prediction of recovery at 6-12 months (i.e. $mRS_{follow-up}<mRS_{discharge}$).

*Statistical analyses*

*Univariate analyses*

Univariate logistic regression analyses were first performed to identify markers able to predict SE outcome. The Benjamini-Hochberg procedure was used to correct for multiple comparisons. The boostrap method was used to estimate the standard errors of $R^2$ (n=1000).

Levels of correlation between quantitative variables and the degree of worsening at discharge ($mRS_{discharge}-mRS_{baseline}$) were obtained with Spearman analyses. Fisher tests were performed to assess whether the frequency distribution of categorical data differed between groups.

*Selection of variables in multivariate analyses*

In order to design scores able to predict SE outcome for all patients, only variables that could be routinely available to clinicians were selected. Firstly, the CSF measures were excluded as lumbar puncture is not systematically performed in SE management (13 variables). Then, measures obtained by UPLC-MS/MS were also excluded, as this method can be only performed in few hospitals (10 variables). Finally, variables with more than 10% of missing data (6 variables), and inter-related variables (9 variables, defined as Spearman's p above 0.80) were discarded. The multivariate analyses were conducted on 29 variables (23 continuous and 6 binary variables).

In this context, the 23 continuous variables are triglycerides (g/L), apolipoprotein B100 (g/L), apolipoprotein E (mg/dL), free cholesterol (g/L), ALAT (alanine aminotransferase) (UI/L), ASAT (aspartate aminotransferase) (UI/L), sodium (mM/L), potassium (mM/L), urea (mM/L), creatinine ($\mu$M/L), total cholesterol (g/L), HDL-cholesterol (g/L), esterified cholesterol (g/L), serum S100B protein (ng/mL), lipoprotein(a) (g/L), progranulin (ng/mL), chloride (mM/L), phospholipids (g/L), serum Neuron specific enolase (ng/mL) and gammaglutamyl transpeptidase (GGT) (UI/L), age (year), duration of SE, functional state before SE (mRSinitial or mRSbaseline as measured by modified Rankin Scale).

The 6 binary (Yes/No) variables are refractoriness of SE, previous history of epilepsy, acute etiology, progressive etiology, remote etiology, cryptogenic (non-assignable) etiology.

Five patients had missing data on some of these 29 variables and were not considered for multivariate ML analysis.

*Machine learning methodology*

A data driven approach was applied, using machine learning (ML) models (support vector machine and logistic regression) to identify markers predictive of SE outcome.

Support Vector Machine (SVM)

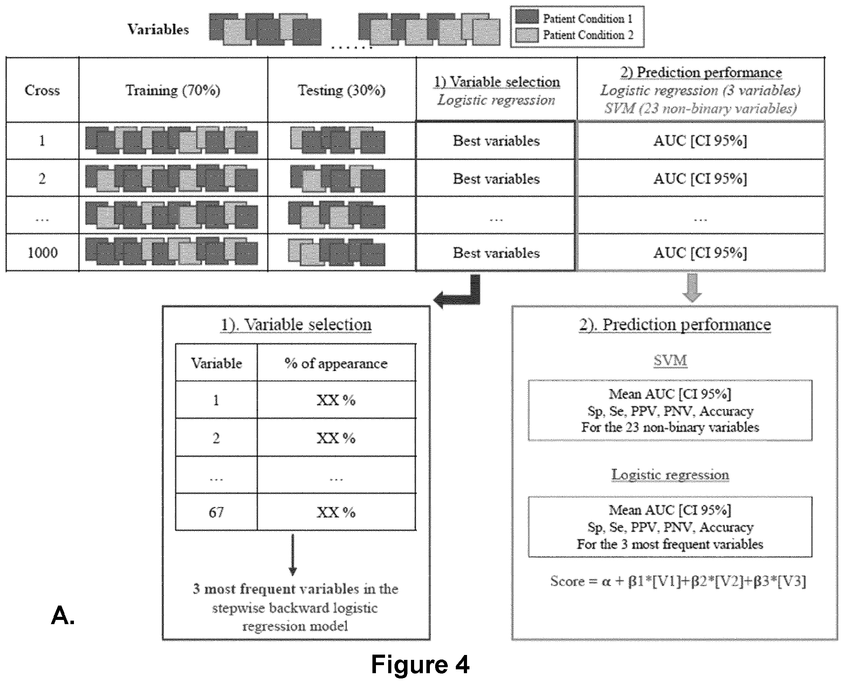

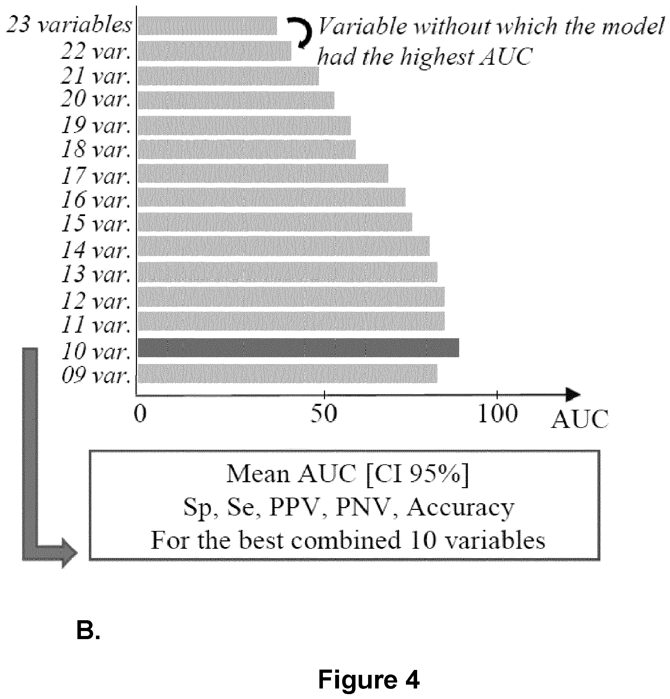

The SVM classifiers are known to be robust to overfitting and work well with complex and high-dimensional datasets.[22] They use a kernel transformation to project input data in a higher dimensional space: input data that cannot be distinguished in the original space may become separable after transformation.[27] Although there are some kernels proposed for binary or categorical variables, most of SVM classifiers are optimized for continuous variables. For this reason, here only the prognosis value of the 23 non-binary variables was evaluated for building the SVM model. There were two stages in building the prediction model (Figure 4.A): a training phase, in which a binary classifier (poor or good outcome, death or survival, recovery or non-recovery) used 70% of observations to learn the model; and then a testing phase, in which the remaining 30% of data were used to evaluate its prediction performance. This cross-validation procedure was used with 1000 folds. The classifiers' performance was also controlled by using a permutation test (n=1000) where class labels are randomly re-assigned.[28]

The most relevant variables were next selected. The most "non-significant" variables were removed one by one by a pruning procedure (Figure 4.B): (i) The area under the receiver operating curve (AUC) values were obtained by cross-validation, after removal of each variable; (ii) the variable without which the model had the highest AUC was removed; and (iii) the procedure was repeated with the remaining variables. A set of "non-relevant" variables that improves the classification after their removal were identified. If the removal procedure further continue with the optimized variables, the classification performance decrease.

Logistic and linear regression models

Logistic regression analysis is currently used to assess relationships between one dependent binary variable and one or more continuous or binary variables. It allowed to construct an index (score) that combined the most important markers. In contrast to SVM, logistic regression models are very sensitive to overfitting. In order to detect reasonable size effects with reasonable power, only one feature per 10 patients was retained. Logistic regression was therefore not used to predict SE mortality and recovery because there were less than 20 patients in both groups.

Here, a linear regression model was also used to identify variables able to predict the degree of worsening at discharge. The validation and reliability of the prediction system were assessed with the Bland-Altman method and the Spearman correlation coefficient.

To identify the most significant variables to assess the poor outcome at discharge, the population was first split into two sets: a training set (70% of observations) and a testing one (the remaining 30% of data) (Figure 4A). A backward stepwise regression procedure with a 1000-fold cross-validation procedure was performed. At each fold, the most significant variables were obtained and the three most frequently found variables were selected (because there were 35 patients in the smallest group). These variables were further used, to build the prediction model (Figure 4.A). Again, a cross-validation procedure (70% of observations were used for the training phase and the remaining 30% for testing) was used with 1000 folds.[28]

Comparison with previous scales

Except the END-IT, the previous scales mostly assessed short-term mortality.[5-8] The scores were not compared to the END-IT because this scale required MRI data for all patients. The prediction performances for poor outcome and mortality were compared to both STESS and mSTESS scales using the better cut-off reported, 3 for STESS score and 4 for mSTESS score, respectively.[6,10,29] The EMSE scale was not used, as some of our patients had SE etiologies, such as auto-immune encephalitis, not covered by this algorithm.

**Example 3. Results**

*Study participants*

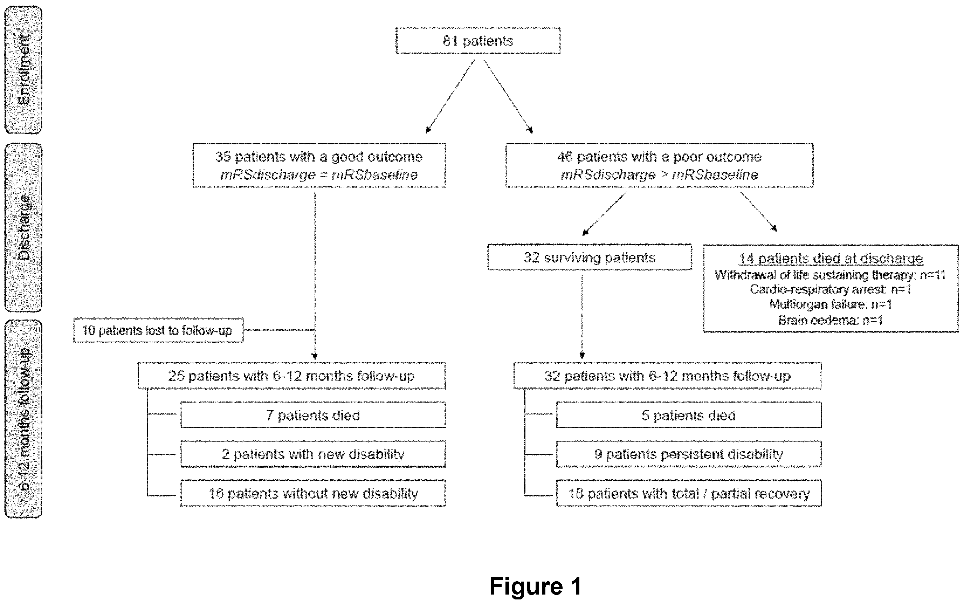

Eighty-one (81) patients with SE (49 men and 32 women, mean age: 50 ($\pm$19) years; mean delay of enrollment (or score calculation) were included after SE onset: 8 ($\pm$15) days) (Figure 1). Fifty-six patients (69%) were initially managed at Pitié-Salpêtrière Hospital while 25 patients (31%) were transferred from another hospital due to uncontrolled SE. Fifty-seven patients (70%) presented with a refractory SE (RSE), defined as a failure of at least two appropriately selected and dosed parenteral medications including a benzodiazepine.[24] Forty-four patients (54%) presented with a super-refractory SE and 29 (36%) with a prolonged super-refractory SE, defined respectively as a RSE that persists for at least 24 hours and 7 days, including ongoing need for anesthetics.[25] The SE etiologies were categorized into four subgroups: acute (29 patients, 36%), remote (24 patients, 30%), progressive (19 patients, 23%), and SE of unknown etiology (9 patients, 11%).[1] Thirty-eight patients (47%) had previously been diagnosed with epilepsy. Thirty-eight patients (47%) had no previous neurological disability ($mRS_{baseline}$=0), while 15 patients (19%) were already dependent before SE ($mRS_{baseline}$ ranging from 3 to 5).

*Outcome prediction at discharge*

Prediction of poor outcome at discharge

Forty-six patients (57%) had a higher $mRS_{discharge}$ score (i.e. poor outcome), when compared with their $mRS_{baseline}$ score (Figure 1). Forty of the 57 patients with RSE (70%) presented with poor outcome after SE compared to only 6 of the 24 patients with a non-refractory SE (25%; *p*=0.049). Patients who had previously been diagnosed with epilepsy had a lower risk to present poor outcome (15 of the 38 patients, 39%) when compared with other patients (31 of the 43 patients, 72%; *p*=0.045).

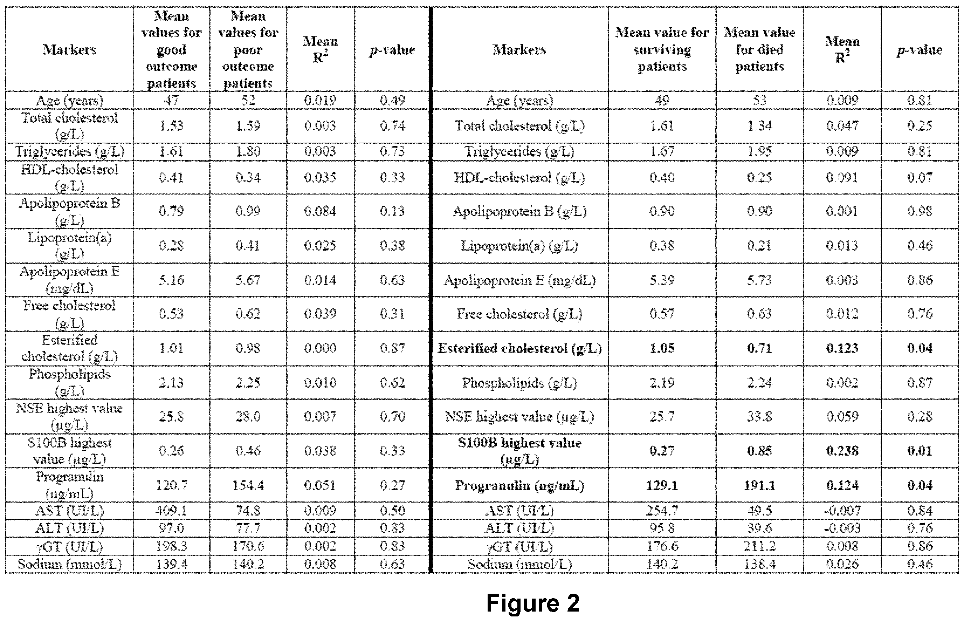

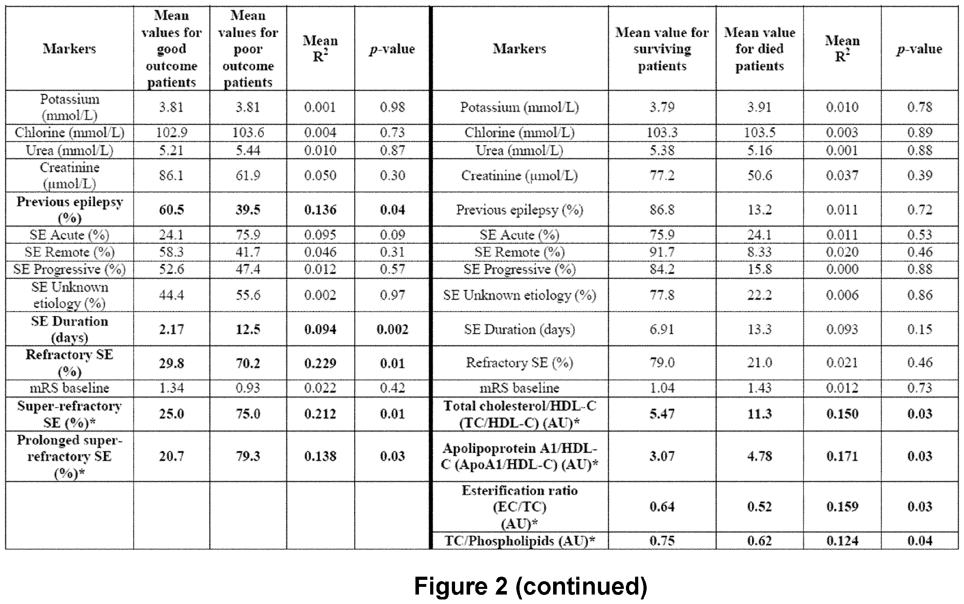

Among the 67 evaluated biomarkers, five clinical markers were significantly different between the 46 patients with poor outcome and the 35 patients for whom SE had no effect on their functional outcome at discharge (Figure 2). None of these biomarkers yielded a sufficient $R^2$ value to be used alone to predict the risk to present poor outcome after SE. Therefore, their outcome predictive potential was assessed by multivariate analyses. The SVM-based predictions using the 23 non-binary variables retained for multivariate analyses failed in most cases (AUC=0.43 [0.23-0.67]). The prediction performance was, however, improved by using the 11 most relevant markers (AUC=0.75 [0.55-0.90], *p*=0.001) and found better when compared with STESS (cut-off at 3, AUC=0.63) and mSTESS (cut-off at 4, AUC=0.53) (Figure 3). The combination of the 11 markers allowed to predict the poor outcome for 77% of the cases (positive predictive value, PPV=0.77, p<0.001). Logistic regression gave similar results to SVM classifier (AUC=0.78 [0.67-0.88], PPV=0.80, *p*<0.001; Figure 3) by using 3 variables: *"Refractory SE",* a binary variable which takes the value of 1 in case of refractory SE or 0 in case of non-refractory SE, *"FC"* the concentration of free cholesterol (g/L) and *"phospholipids"* the concentration of phospholipids (g/L).

Prediction of the degree of worsening at discharge

Forty-six of the 81 patients (57%) had poor outcome after SE. The difference between their $mRS_{baseline}$ and their $mRS_{discharge}$ scores was of 1 for 13 patients (28%), 2 for 6 patients (13%), 3 for 7 patients (15%), 4 for 7 patients (15%), 5 for 9 patients (20%) and 6 for 4 patients (9%).

Among the 67 evaluated biomarkers, 17 clinical and biochemical markers were significantly correlated with the difference "$mRS_{discharge}$-$mRS_{baseline}$" (Table 2). By linear regression analysis, we identified the three most relevant variables to assess the degree of disability: the highest serum value of S100beta protein (S100B) (ng/mL), the $mRS_{baseline}$ and the creatinine value ($\mu$mol/L), by backward analysis, as disclosed above.

Table 2: Estimation of the degree of worsening at discharge

| Markers | Spearman's r estimate | p-value |
|---|---|---|
| Age (years) | -0.068 | 0.87 |
| Total cholesterol (g/L) | -0.303 | 0.14 |
| Triglycerides (g/L) | 0.377 | 0.04 |
| HDL-cholesterol (g/L) | -0.482 | 0.01 |
| Apolipoprotein B (g/L) | 0.092 | 0.78 |
| Lipoprotein(a) (g/L) | -0.162 | 0.55 |
| Apolipoprotein E (mg/dL) | 0.372 | 0.05 |
| Free cholesterol (g/L) | 0.156 | 0.55 |
| Esterified cholesterol (g/L) | -0.474 | 0.01 |
| Phospholipids (g/L) | 0.074 | 0.85 |

(continued)

| Markers | Spearman's r estimate | p-value |
| --- | --- | --- |
| NSE highest value ($\mu$g/L) | 0.264 | 0.22 |
| S100B highest value ($\mu$g/L) | 0.510 | 0.01 |
| Progranulin (ng/mL) | 0.464 | 0.01 |
| AST (UI/L) | 0.273 | 0.19 |
| ALT (UI/L) | 0.214 | 0.34 |
| gGT (UI/L) | 0.177 | 0.48 |
| Sodium (mmol/L) | -0.128 | 0.62 |
| Potassium (mmol/L) | 0.097 | 0.78 |
| Chlorine (mmol/L) | -0.004 | 0.99 |
| Urea (mmol/L) | 0.007 | 0.99 |
| Creatinine ($\mu$mol/L) | -0.377 | 0.05 |
| Previous epilepsy (%) | -0.107 | 0.03 |
| SE Acute (%) | 0.151 | 0.01 |
| SE Remote (%) | -0.096 | 0.03 |
| SE Progressive (%) | -0.029 | 0.62 |
| SE Unknown etiology (%) | -0.026 | 0.58 |
| SE Duration (days) | 0.543 | <0.001 |
| Refractory SE (%) | 0.012 | 0.87 |
| mRS baseline | -0.415 | 0.02 |
| Prolonged super-refractory status epilepticus (%) | 0.121 | 0.02 |
| Apolipoprotein A1 (g/L)* | -0.584 | 0.02 |
| Esterification ratio (EC/TC) (AU)* | -0.496 | 0.01 |
| TC/Phospholipids (AU)* | -0.477 | 0.01 |
| GCS at enrollment* | -0.421 | 0.02 |

The correlation between the markers and the difference between $mRS_{discharge}$ and $mRS_{baseline}$ was assessed with Spearman analysis.

The variables in bold were significantly associated with the degree of worsening at discharge.

*Markers not considered for multivariate analyses.

Abbreviations: ALT = Alanine Aminotransferase; AST = Aspartate Aminotransferase; ; AU = Arbitrary Unit; ICU = Intensive Care Unit; GCS = Glasgow Coma Scale; mRS = modified Rankin Score; NSE = Neuron Specific Enolase; SE = Status Epilepticus

The Bland-Altman analysis reported a 95% agreement between -2.5 to 2.3 with a bias of -0.14 between the real ($mRS_{discharge}$-$mRS_{baseline}$) and the predicted score. Moreover, significant correlation coefficients between both measurements are revealed in all states (Spearman's p=0.724, $p<0.001$).

Prediction of mortality at discharge

Fourteen patients died at hospital discharge (mean delay after SE onset, 47 ($\pm$40) days), mostly after the withdrawal of life sustaining therapy (11 patients, 79%) (Figure 1). Twelve of the 57 patients with RSE (21%) died at hospital discharge compared to 2 of the 24 patients (8.3%) with non-refractory SE (p=0.34). Nine of the 29 patients with prolonged super-refractory SE (31%) died at hospital discharge compared to only 5 of the 52 patients with non-prolonged super-refractory SE (9.6%) (p=0.074). The risk to die was not significantly higher for patients with RSE or prolonged super-refractory SE due to lack of statistical power. Half of the 14 died patients presented with SE of acute etiology and five of them had previously been diagnosed with epilepsy (36%).

Among the 67 evaluated biomarkers, seven biochemical markers were significantly different between the 14 died patients and the 67 surviving patients (Figure 2). Nevertheless, none of these biomarkers had a sufficient $R^2$ value to be used alone to predict the risk of death after SE. Therefore, we assessed their outcome predictive potential by multivariate analyses. The SVM-based predictions using the 23 non-binary variables retained for multivariate analyses failed in most cases (AUC=0.37 [0.16-0.70]). However, the prediction performance was improved using the 10 most relevant markers (AUC=0.73 [0.54-0.91], p=0.001), which was clearly better than those obtained with STESS (cut-off at 3, AUC=0.56) and mSTESS (cut-off at 4, AUC=0.62) (Figure 3). The combination of the 10 most discriminant variables allowed to predict the death after SE in over 40% of cases (PPV=0.41, p=0.018). As the number of observations was unequal in our two groups, we also computed the F1 score, which is a more appropriate metrics for imbalanced scenarios, and defined as the harmonic mean of precision (PPV) and recall (sensitivity).[30] For the 10 most relevant markers, the F1 score was of 0.53 [0.32-0.89]; a higher value than those of STESS (0.29) and mSTESS (0.36) scales.

*Outcome prediction at long-term*

Prediction of recovery at long-term

All 32 surviving patients with poor outcome after SE underwent a follow-up neurological evaluation at 6-12 months. Eighteen patients (56%) showed partial or total recovery of neurologic symptoms (Figure 1).

None of the 67 evaluated biomarkers was significantly different between the 18 patients who recovered and the remaining 14 patients. Nevertheless, we assessed their outcome predictive potential by multivariate analyses. The SVM-based predictions using the 23 non-binary variables retained for multivariate analyses had a moderate ability (AUC=0.57 [0.20-0.90]) to predict the patient evolution. Nevertheless, the prediction performance was improved using the 12 most relevant markers (AUC=0.86 [0.60-1.0], p<0.001). SVM models were able to predict the recovery for 91% of the cases (PPV=0.91, p=0.001). Moreover, they were able to predict which patients will have persistent disability in 83% of the cases (negative predictive value, NPV=0.83, p=0.002).

**Example 4. Discussion**

To better manage SE, it is important to have tools that enable to accurately predict both poor and good outcomes, at discharge and at long-term. Four prognosis scales have been proposed in the last fifteen years. Nevertheless, none can be used to follow all SE patients over time: STESS and mSTESS scales can be applied for all SE patients but they are built only on pre-hospitalized data and so cannot be used to follow the evolution of the patient in ICU; EMSE algorithm covered only some SE etiologies; and END-IT scale requires MRI data. [5-8] Here, using a cohort of 81 patients and applying ML methods, it was found that ML methods can predict patient's outcome for all hospitalized SE patients and at different time points.

*Outcome prediction at discharge*

In agreement with previous reports, we found a higher risk of poor outcome (i.e. death or worsening of clinical conditions) for patients with RSE, higher SE duration and a lower risk for patients who had previously been diagnosed with epilepsy.[2,3,5,8,31]

Two clinico-biological tools able are herein described to predict the outcome at discharge. Conversely to END-IT which required MRI data,[7] both these scores can be applied for all SE patients.

The first SVM model retained 11 variables to predict the outcome at discharge. The selected variables can be obtained quickly and reflected non-neurologic organ failure (hepatic dysfunction: total cholesterol, HDL-cholesterol, lipoprotein(a), aspartate aminotransferase; renal dysfunction: urea, creatinine; systemic dysfunction: potassium, chloride),[32] the inflammation process induced by SE (S100B, progranulin),[17] and the disease severity highlighted by the SE duration before enrollment.[31] This model accurately predicted the outcome (AUC 0.75 [0.55-0.90]). It resulted in a 19% improvement in AUC over the STESS and 42% over the mSTESS. This model was also accurate to predict which patients will have a good outcome at discharge (NPV=0.76).

The logistic regression model made it possible to construct a score that combined the 3 most important markers: a binary variable (RSE) and two continuous variables (free cholesterol, FC and phospholipids levels). Patients with RSE were more likely to have a poor outcome at discharge.[32] Similarly, patients with higher FC levels had poor outcome more frequently. It was previously found that SE patients had higher FC levels when compared with control or epileptic patients.[21] The accumulation of FC in neuronal cells was found responsible to neuronal death.[19] This can lead to neurocognitive sequels and may explain why patients with higher FC levels had poorer prognosis. Conversely, patients with higher phospholipids levels presented with a better outcome. Phospholipids composed cellular membranes and are essential for the proper functioning of the membrane-bound proteins.[33] A decrease in phospholipids levels may disturb the properties of cellular membranes and induce a conformational change of the membrane.[34] It may affect the activity of the $Ca^{2+}$-ATPase, the $Na^+,K^+$-ATPase or also the sterol-regulatory-element-binding protein that would induce cellular dysfunctions and subsequent sequels.[34] Nevertheless, all phospholipids were analyzed simultaneously and this result may hide different trends from the various subtypes of phospholipids. The logistic regression model accurately predicted SE outcome (AUC 0.78 [0.67-0.88]) and resulted in a 24% improvement in AUC over the STESS and 47% over the mSTESS. The results were similar as those obtained with the SVM model to predict the poor outcome but the performances were lower to predict the good outcome (NPV=0.56 vs NPV=0.76). These two ML models (SVM and logistic regression) may allow to easily evaluate the impact of a new neuroprotective or antiepileptic therapeutic on the outcome and the evolution of the patient over time.

It is believed that this provides for the first time a clinico-biological score able to predict the degree of worsening induced by SE. The approach herein described is particularly relevant to better manage SE by providing information to physicians and families. The ML model combined three variables: the $mRS_{baseline}$, the S100B and the creatinine levels. Patients with lower $mRS_{baseline}$ are more likely to present with higher degree of worsening at discharge. This result may be explained as 22% of our patients presented with a New-Onset Refractory Status Epilepticus (NORSE), which occurs in patients without preexisting relevant neurologic disorder,[25] often young and without other medical history. These patients had the poorer outcome and the longer stay duration in ICU. They are often dependent in the first months after SE due to their cognitive sequels and their inability to walk alone following critical illness neuropathy. The high percentage of NORSE patients in our cohort can be explained as most of our patients were enrolled in a tertiary unit, specialized in the management of super-refractory SE. Increased serum S100B levels were found after an isolated seizure but this biomarker was not previously studied in human SE.[16] The S100B is produced by astrocytes and Schwann cells. At micromolar levels, the S100B have toxic effects by inducing apoptosis and stimulating the expression of pro-inflammatory cytokines.[18] This may explain why higher S100B levels were associated with a higher degree of worsening. Patients with lower creatinine levels presented with a higher degree of worsening. This may reflect the muscular atrophy induced by prolonged ICU stay, with a higher risk of critical illness neuropathy making patients dependent on walking with a $mRS_{discharge}$ above 3.

In contrast to previous studies, a significantly higher risk of mortality was not found, for older patients, patients with an acute SE or with a RSE.[3,5,8,31] This can be explained by an enrollment bias: most of the patients were enrolled in the neuro ICU of Pitié-Salpêtrière Hospital, a tertiary unit, specialized in the management of super-refractory SE. Super-refractory SE can be induced by acute immune disorders and mostly concern younger patients.[3,35]

The SVM model using the 10 most relevant markers was able to predict with a good accuracy the risk of mortality (AUC=0.73 [0.54-0.91]). It resulted in a 30% improvement in AUC over the STESS and 18% over the mSTESS. The 10 variables used by the SVM classifier are routinely available, potentially allowing for easier integration in ICU. They reflected non-neurologic organ failure (hepatic dysfunction: triglycerides, apolipoproteins B and E, free cholesterol, alanine aminotransferase, aspartate aminotransferase; renal dysfunction: urea, creatinine; systemic dysfunction: sodium, potassium), of which a part is known to be associated with the risk of SE and its prognosis.[36,37] The model allowed also to predict a positive outcome: the negative predictive value (NPV) of 0.94 (p=0.002) means that a negative test is almost an indicator of survival. The model seems to show a lower efficiency in predicting mortality when compared with the first publication using EMSE.[8] EMSE considers the SE etiology, while SVM classifier does not allow to simultaneously integrate binary and continuous variables. Nevertheless, less favorable results were reported thereafter with EMSE.[38]

*Outcome prediction at long-term*

It is herein provided for the first-time a tool allowing the prediction of recovery at long-term. It is particularly relevant in the management of SE: a high probability of recovery at long-term may prompt clinicians to continue anesthesia for an extended period of time before deciding to discontinue life sustaining therapies. In addition, it is also relevant to provide accurate long-term prognostication to families. The SVM model retained the 12 most relevant variables to predict accurately the recovery (AUC 0.86 [0.60-1.0]). The selected variables reflected non-neurologic organ failure (hepatic dysfunction: apolipoprotein B, free cholesterol, gamma GT; renal dysfunction: urea, creatinine; systemic dysfunction: sodium, chloride),[32] the brain injury induced by SE (highest serum Neuron Specific Enolase value),[12] and the severity of the disease highlighted by the SE duration.[31] The age and the $mRS_{baseline}$ are also retained by the algorithm: younger patients without medical history may recover more easily. The last variable was the level of phospholipids. It can be hypothesized that higher phospholipids levels may induce lower cellular dysfunctions and that these disturbances may be reversible.

There are three main findings in this report. Firstly, the ML models predict the functional outcome and the mortality at discharge better than the two previous scales, STESS and mSTESS, and the ML models can be applied for all hospitalized SE patients. Secondly, the ML models allow to estimate the degree of worsening induced by SE, which can help to adapt therapeutics. Finally, the ML models can also predict the recovery at long-term when including variables obtained upon admission.

The study was conducted in a single cohort of patients who were enrolled in a single hospital. The results thus may be refined, using a larger cohort or patients from various hospitals. Patients presented with various SE etiologies and were enrolled at different time points after SE onset. It is also to be noted that the prediction of mortality at discharge has to be interpreted with caution as almost 80% of the patients died after the withdrawal of life sustaining therapies. However, despite the fact that the selected variables and the performances of the disclosed models might have been different in other centers with other protocols, the results herein reported show that it is possible to obtain high quality models using machine learning, or linear regression, using easily measurable variables. To minimize the model overfitting, a 1000-fold cross validation procedure and a 1000-fold permutation test to control the classifier's performance were used. This study is the first that provides an efficient framework for the prediction of functional outcome, mortality at discharge, and recovery at long-term. The described tools integrate also biochemical data to reflect pathophysiological mechanisms involved in SE excitotoxicity and consequences. Contrary to previous scales, these ML tools can be applied for all hospitalized SE patients, enabling to monitor SE patients over time, to follow the impact of a new therapeutic, or to define a targeted, sufficiently homogenous, population for further clinical trials in order to permit precise estimation of treatment effect. To address the issue of their clinical liability, ML models can be highly operable in mobile devices, which would facilitate their use in routine ICU setting.[40]

In the future, the model can be expanded to include imaging or electrographic biomarkers to improve the performances.

## References

1. Trinka E, Kälviäinen R. 25 years of advances in the definition, classification and treatment of status epilepticus. Seizure. 2017;44:65-73.

2. Leitinger M, Trinka E, Giovannini G, et al. Epidemiology of status epilepticus in adults: A population-based study on incidence, causes, and outcomes. Epilepsia. 2019;60:53-62.

3. Alkhachroum A, Der-Nigoghossian CA, Rubinos C, Claassen J. Markers in Status Epilepticus Prognosis. J Clin Neurophysiol. 2020;37:422-428.

4. Kantanen A-M, Reinikainen M, Parviainen I, Kälviäinen R. Long-term outcome of refractory status epilepticus in adults: A retrospective population-based study. Epilepsy Res. 2017;133:13-21.

5. Rossetti AO, Logroscino G, Bromfield EB. A clinical score for prognosis of status epilepticus in adults. Neurology. 2006;66:1736-1738.

6. Gonzalez-Cuevas M, Santamarina E, Toledo M, et al. A new clinical score for the prognosis of status epilepticus in adults. Eur J Neurol. 2016;23:1534-1540.

7. Gao Q, Ou-Yang T, Sun X, et al. Prediction of functional outcome in patients with convulsive status epilepticus: the END-IT score. Crit Care. 2016;20:46.

8. Leitinger M, Holler Y, Kalss G, et al. Epidemiology-based mortality score in status epilepticus (EMSE). Neurocrit Care. 2015;22:273-282.

9. Yuan F, Gao Q, Jiang W. Prognostic scores in status epilepticus-a critical appraisal. Epilepsia. 2018;59 Suppl 2:170-175.

10. Rossetti AO, Logroscino G, Milligan TA, Michaelides C, Ruffieux C, Bromfield EB. Status Epilepticus Severity Score (STESS): a tool to orient early treatment strategy. J Neurol. 2008;255:1561-1566.

11. Hanin A, Lambrecq V, Denis JA, et al. Cerebrospinal fluid and blood biomarkers of status epilepticus. Epilepsia. 2020;61:6-18.

12. DeGiorgio CM, Gott PS, Rabinowicz AL, Heck CN, Smith TD, Correale JD. Neuron-specific enolase, a marker of acute neuronal injury, is increased in complex partial status epilepticus. Epilepsia. 1996;37:606-609.

13. DeGiorgio CM, Correale JD, Gott PS, et al. Serum neuron-specific enolase in human status epilepticus. Neurology. 1995;45:1134-1137.

14. DeGiorgio CM, Heck CN, Rabinowicz AL, Gott PS, Smith T, Correale J. Serum neuron-specific enolase in the major subtypes of status epilepticus. Neurology. 1999;52:746-749.

15. Correale J, Rabinowicz AL, Heck CN, Smith TD, Loskota WJ, DeGiorgio CM. Status epilepticus increases CSF levels of neuron-specific enolase and alters the blood-brain barrier. Neurology. 1998;50:1388-1391.

16. Freund Y, Bloom B, Bokobza J, et al. Predictive value of S100-B and copeptin for outcomes following seizure: the BISTRO International Cohort Study. PLoS ONE. 2015;10:e0122405.

17. Zhu S, Tai C, Petkau TL, et al. Progranulin promotes activation of microglia/macrophage after pilocarpine-induced status epilepticus. Brain Research. 2013; 1530:54-65.

18. Sen J, Belli A. S100B in neuropathologic states: the CRP of the brain? J Neurosci Res. 2007;85:1373-1380.

19. Chali F, Djelti F, Eugene E, et al. Inhibiting cholesterol degradation induces neuronal sclerosis and epileptic activity in mouse hippocampus. Eur J Neurosci. 2015;41: 1345-1355.

20. Chali F, Milior G, Marty S, et al. Lipid markers and related transcripts during excitotoxic neurodegeneration in kainate-treated mice. Eur J Neurosci. 2019;50: 1759-1778.

21. Hanin A, Baudin P, Demeret S, et al. Disturbances of brain cholesterol metabolism: A new excitotoxic process associated with status epilepticus. Neurobiol Dis. 2021;154:105346.

22. Chaudhry F, Hunt RJ, Hariharan P, et al. Machine Learning Applications in the Neuro ICU: A Solution to Big Data Mayhem? Front Neurol. 2020; 11:554633.

23. Elm E von, Altman DG, Egger M, et al. The Strengthening the Reporting of Observational Studies in Epidemiology (STROBE) Statement: Guidelines for Reporting Observational Studies. PLOS Medicine. Public Library of Science; 2007;4:e296.

24. Trinka E, Cock H, Hesdorffer D, et al. A definition and classification of status epilepticus--Report of the ILAE Task Force on Classification of Status Epilepticus. Epilepsia. 2015;56:1515-1523.

25. Hirsch LJ, Gaspard N, van Baalen A, et al. Proposed consensus definitions for new-onset refractory status epilepticus (NORSE), febrile infection-related epilepsy syndrome (FIRES), and related conditions. Epilepsia. 2018;59:739-744.

26. Bruno A, Shah N, Lin C, et al. Improving modified Rankin Scale assessment with a simplified questionnaire. Stroke. 2010;41:1048-1050.

27. Noble WS. What is a support vector machine? Nature Biotechnology. 2006;24.

28. Ojala M, Garriga G. Permutation Tests for Studying Classifier Performance. J Mach Learn Res. 2010;11:1833-1863.

29. Giovannini G, Monti G, Tondelli M, et al. Mortality, morbidity and refractoriness prediction in status epilepticus: Comparison of STESS and EMSE scores. Seizure. 2017;46:31-37.

30. Saito T, Rehmsmeier M. The precision-recall plot is more informative than the ROC plot when evaluating binary classifiers on imbalanced datasets. PLoS One. 2015;10:e0118432.

31. Marawar R, Basha M, Mahulikar A, Desai A, Suchdev K, Shah A. Updates in Refractory Status Epilepticus. Crit Care Res Pract. 2018;2018:9768949.

32. Ciurans J, Grau-López L, Jimenez M, Fumanal A, Misis M, Becerra JL. Refractory status epilepticus: Impact of baseline comorbidity and usefulness of STESS and EMSE scoring systems in predicting mortality and functional outcome. Seizure. 2018;56:98-103.

33. Adibhatla RM, Hatcher JF. Altered lipid metabolism in brain injury and disorders. Subcell Biochem. 2008;49:241-268.

34. Maxfield FR, Tabas I. Role of cholesterol and lipid organization in disease. Nature. 2005;438:612-621.

35. Gaspard N, Hirsch LJ, Sculier C, et al. New-onset refractory status epilepticus (NORSE) and febrile infection-related epilepsy syndrome (FIRES): State of the art and perspectives. Epilepsia. 2018;59:745-752.

36. Sonneville R, Mariotte E, Neuville M, et al. Early-onset status epilepticus in patients with acute encephalitis. Medicine (Baltimore). 2016;95:e4092.

37. Gaspard N, Foreman BP, Alvarez V, et al. New-onset refractory status epilepticus: Etiology, clinical features, and outcome. Neurology. 2015;85:1604-1613.

38. Pacha MS, Orellana L, Silva E, et al. Role of EMSE and STESS scores in the outcome evaluation of status epilepticus. Epilepsy Behav. 2016;64:140-142.

39. Hutson M. Artificial intelligence faces reproducibility crisis. Science. 2018;359:725-726.

40. The Lancet Respiratory Medicine. Opening the black box of machine learning. Lancet Respir Med. 2018;6:801.

41. Wilson et al, 2005, Stroke. 36 (4): 777-781

42. Fernández A, García S, Galar M, Prati RC, Krawczyk B, Herrera F. Learning from imbalanced data sets. New York, NY: Springer International Publishing; 2018

## Claims

1. An *in vitro* method for prognosis of the outcome of status epilepticus for a patient, comprising:

   a. Providing the values of at least three markers, including at least one biological marker,
   b. Combining the values, optionally normalized, provided in a) in order to obtain an end value

   wherein the end value is indicative of the outcome of status epilepticus.

2. The method of claim 1, wherein the combination is performed in a processing device *via* a configured artificial machine learning classifier, which generate classes of outcome of the status of the patient as the end value.

3. The method of claim 2, wherein the machine learning classifier is support vector machine, in particular a two-classes support vector machine.

4. The method of claim 1, wherein the combination is performed through a logistic regression function, which generates an end value that is compared to a reference value to predict the outcome of status epilepticus.

5. The method of any one of claims 1 to 4, wherein the at least one biological marker is selected in the group consisting of triglycerides (g/L), apolipoprotein B100 (g/L), apolipoprotein E (mg/dL), free cholesterol (g/L), ALAT (alanine aminotransferase) (UI/L), ASAT (aspartate aminotransferase) (UI/L), sodium (mM/L), potassium (mM/L), urea (mM/L), creatinine ($\mu$M/L), total cholesterol (g/L), HDL-cholesterol (g/L), serum S100B protein (ng/mL), lipoprotein(a) (g/L), progranulin (ng/mL), chloride (mM/L), phospholipids (g/L), serum Neuron specific enolase (ng/mL) and gammaglutamyl transpeptidase (GGT) (UI/L).

6. The method of any one of claims 1 to 5, wherein the age of the patient is combined with the value of the biological marker in order to obtain the end value.

7. The method of any one of claims 1 to 6, wherein at least one marker associated with the clinical condition of the patient is combined with the value of the biological marker in order to obtain the end value.

8. The method of claim 7, wherein the value associated with the clinical condition of the patient is selected in the group consisting of duration of status epilepticus (days), initial Rankin (functional state of the patient before status epilepticus), and status refractoriness (1 is case of refractory status epilepticus, 0 in case of non-refractory status epilepticus).

9. The method of any one of claims 1 to 8, wherein

   a. the outcome is the risk of death of the patient in intensive care unit, and wherein the markers are triglycerides, apolipoprotein B100 (g/L), apolipoprotein E (mg/dL), free cholesterol (g/L), ALAT (alanine aminotransferase) (UI/L), ASAT (aspartate aminotransferase) (UI/L), sodium (mM /L), potassium (mM /L), urea (mM /L), creatinine ($\mu$M/L), or
   b. the outcome is the risk of poor outcome (i.e. death or worsening of clinical conditions) on discharge from the intensive care unit, and wherein the markers are total cholesterol (g/L), HDL-cholesterol (g/L), lipoprotein(a) (g/L), S100B highest serum value (ng/mL), progranulin (ng/mL), ASAT (UI/L), potassium (mM /L), chloride (mM /L), urea (mM/L), creatinine ($\mu$M/L), duration of status epilepticus before evaluation (days), or
   c. the outcome is the risk of poor outcome (i.e. death or worsening of clinical conditions) on discharge from the intensive care unit, and wherein the markers are status refractoriness (1 is case of refractory status epilepticus, 0 in case of non-refractory status epilepticus), free cholesterol (g/l) and phospholipids (g/l), or
   d. the outcome is the degree of worsening expected at discharge from the intensive care unit, and wherein the markers are S100B highest serum value (ng/ml) during status epilepticus, initial Rankin (functional state of the patient before status epilepticus) and creatinine ($\mu$M/l), or
   e. the outcome is the remote recovery from status epilepticus, and wherein the markers are age (years), apolipoprotein B100 (g/L), free cholesterol (g/L), phospholipids (g/L), maximal value of serum Neuron specific enolase (ng/mL), GGT (UI/L), sodium (mM/L), chloride (mM/L), urea (mM/L), creatinine ($\mu$M/L), duration of status epilepticus (days) and initial Rankin.

10. The method of any one of claims 1 to 9, which is computer implemented.

11. The method of any one of claims 1 to 10, comprising:

   a. receiving, by a processing device, signal data representing values of at least three markers, including at least one biological marker, and optionally age of the patient,
   b. analyzing said signal data, by the processing device *via* a configured artificial machine learning classifier, in order to generate an output indicative of the outcome of status epilepticus.

12. A method for prognosis of the outcome of status epilepticus for a patient, comprising:

a. Obtaining values of at least three markers, including at least one biological marker, and optionally age of the patient;

b. Optionally pre-processing such values to extract their mean and set their variance equal to one, in order to obtain normalized values

c. inputting such values or normalized values to a machine learning classifier, in particular a support vector machine, configured to process the values and provide an output associated with status epilepticus,

d. obtaining an output from the machine learning classifier, wherein the output is indicative of the outcome of status epilepticus.

13. The method of claim 16, wherein the values used in are sent to a remote server for pre-processing and/or processing by the machine learning classifier and wherein the output is sent to a physician.

14. A method for producing a machine learning classifier capable of prognosis of the outcome of status epilepticus for a patient, comprising:

   a. storing in an electronic database patient data comprising

      i. input data consisting of values, optionally normalized, of at least three markers, including at least one biological marker, and optionally age of the patient, and
      ii. classes corresponding to different outcomes of status epilepticus for the patient;

   b. providing a machine learning system; and
   c. training the machine learning system using the patient data, such that the machine learning system is trained to assign input data to the appropriate class, so that it can produce a prognosis of the outcome of status epilepticus for a patient when exposed to input data from the patient.

15. The method of claim 18, wherein the classification system is a two-classes support-vector machine classifier, or a neural network, preferably a convolutional neural network.

16. The method of any one of claims 14 or 15, where the patient is assigned to one class by the following rules:

   a. For the good/poor outcome classification.

      i. "Good outcome" class when clinical conditions of patient on discharge are equal than initial clinical conditions
      ii. "Poor outcome" class when clinical conditions of patient on discharge are worse than initial clinical conditions

   b. For the death/survival classification:

      i. "Death" class when patient dies
      ii. "Survival" class when patient survives

   c. For the Recovery/Non recovery classification:

      i. "Recovery" class when clinical conditions of patient at long term are better than clinical conditions at discharge
      ii. "Non recovery" class when clinical conditions of patient at long term are equal or worse than clinical conditions at discharge

17. A device comprising:

   a. at least one interface for entering patient data comprising values of at least three markers, including at least one biological marker, and optionally age of the patient, and/or age of the patient;
   b. a processing unit comprising at least one processor; and
   c. at least one non-transitory computer-readable medium comprising program instructions that, when executed by the at least one processor, causes the device to:

i. process the patient data, if necessary to standardize the unities of the values;

ii. provide patient data, optionally standardized, to a machine learning classifier,

thereby obtaining an output from the machine learning classifier, said output being a prognosis of the outcome of status epilepticus for a patient when exposed to input data from the patient.

Figure 1

| Markers | Mean values for good outcome patients | Mean values for poor outcome patients | Mean $R^2$ | $p$-value | Markers | Mean value for surviving patients | Mean value for died patients | Mean $R^2$ | $p$-value |
|---|---|---|---|---|---|---|---|---|---|
| Age (years) | 47 | 52 | 0.019 | 0.49 | Age (years) | 49 | 53 | 0.009 | 0.81 |
| Total cholesterol (g/L) | 1.53 | 1.59 | 0.003 | 0.74 | Total cholesterol (g/L) | 1.61 | 1.34 | 0.047 | 0.25 |
| Triglycerides (g/L) | 1.61 | 1.80 | 0.003 | 0.73 | Triglycerides (g/L) | 1.67 | 1.95 | 0.009 | 0.81 |
| HDL-cholesterol (g/L) | 0.41 | 0.34 | 0.035 | 0.33 | HDL-cholesterol (g/L) | 0.40 | 0.25 | 0.091 | 0.07 |
| Apolipoprotein B (g/L) | 0.79 | 0.99 | 0.084 | 0.13 | Apolipoprotein B (g/L) | 0.90 | 0.90 | 0.001 | 0.98 |
| Lipoprotein(a) (g/L) | 0.28 | 0.41 | 0.025 | 0.38 | Lipoprotein(a) (g/L) | 0.38 | 0.21 | 0.013 | 0.46 |
| Apolipoprotein E (mg/dL) | 5.16 | 5.67 | 0.014 | 0.63 | Apolipoprotein E (mg/dL) | 5.39 | 5.73 | 0.003 | 0.86 |
| Free cholesterol (g/L) | 0.53 | 0.62 | 0.039 | 0.31 | Free cholesterol (g/L) | 0.57 | 0.63 | 0.012 | 0.76 |
| Esterified cholesterol (g/L) | 1.01 | 0.98 | 0.000 | 0.87 | **Esterified cholesterol (g/L)** | **1.05** | **0.71** | **0.123** | **0.04** |
| Phospholipids (g/L) | 2.13 | 2.25 | 0.010 | 0.62 | Phospholipids (g/L) | 2.19 | 2.24 | 0.002 | 0.87 |
| NSE highest value (μg/L) | 25.8 | 28.0 | 0.007 | 0.70 | NSE highest value (μg/L) | 25.7 | 33.8 | 0.059 | 0.28 |
| S100B highest value (μg/L) | 0.26 | 0.46 | 0.038 | 0.33 | **S100B highest value (μg/L)** | **0.27** | **0.85** | **0.238** | **0.01** |
| Progranulin (ng/mL) | 120.7 | 154.4 | 0.051 | 0.27 | **Progranulin (ng/mL)** | **129.1** | **191.1** | **0.124** | **0.04** |
| AST (UI/L) | 409.1 | 74.8 | 0.009 | 0.50 | AST (UI/L) | 254.7 | 49.5 | -0.007 | 0.84 |
| ALT (UI/L) | 97.0 | 77.7 | 0.002 | 0.83 | ALT (UI/L) | 95.8 | 39.6 | -0.003 | 0.76 |
| γGT (UI/L) | 198.3 | 170.6 | 0.002 | 0.83 | γGT (UI/L) | 176.6 | 211.2 | 0.008 | 0.86 |
| Sodium (mmol/L) | 139.4 | 140.2 | 0.008 | 0.63 | Sodium (mmol/L) | 140.2 | 138.4 | 0.026 | 0.46 |

# Figure 2

| Markers | Mean values for good outcome patients | Mean values for poor outcome patients | Mean $R^2$ | $p$-value | Markers | Mean value for surviving patients | Mean value for died patients | Mean $R^2$ | $p$-value |
|---|---|---|---|---|---|---|---|---|---|
| Potassium (mmol/L) | 3.81 | 3.81 | 0.001 | 0.98 | Potassium (mmol/L) | 3.79 | 3.91 | 0.010 | 0.78 |
| Chlorine (mmol/L) | 102.9 | 103.6 | 0.004 | 0.73 | Chlorine (mmol/L) | 103.3 | 103.5 | 0.003 | 0.89 |
| Urea (mmol/L) | 5.21 | 5.44 | 0.010 | 0.87 | Urea (mmol/L) | 5.38 | 5.16 | 0.001 | 0.88 |
| Creatinine (µmol/L) | 86.1 | 61.9 | 0.050 | 0.30 | Creatinine (µmol/L) | 77.2 | 50.6 | 0.037 | 0.39 |
| **Previous epilepsy (%)** | **60.5** | **39.5** | **0.136** | **0.04** | Previous epilepsy (%) | 86.8 | 13.2 | 0.011 | 0.72 |
| SE Acute (%) | 24.1 | 75.9 | 0.095 | 0.09 | SE Acute (%) | 75.9 | 24.1 | 0.011 | 0.53 |
| SE Remote (%) | 58.3 | 41.7 | 0.046 | 0.31 | SE Remote (%) | 91.7 | 8.33 | 0.020 | 0.46 |
| SE Progressive (%) | 52.6 | 47.4 | 0.012 | 0.57 | SE Progressive (%) | 84.2 | 15.8 | 0.000 | 0.88 |
| SE Unknown etiology (%) | 44.4 | 55.6 | 0.002 | 0.97 | SE Unknown etiology (%) | 77.8 | 22.2 | 0.006 | 0.86 |
| **SE Duration (days)** | **2.17** | **12.5** | **0.094** | **0.002** | SE Duration (days) | 6.91 | 13.3 | 0.093 | 0.15 |
| **Refractory SE (%)** | **29.8** | **70.2** | **0.229** | **0.01** | Refractory SE (%) | 79.0 | 21.0 | 0.021 | 0.46 |
| mRS baseline | 1.34 | 0.93 | 0.022 | 0.42 | mRS baseline | 1.04 | 1.43 | 0.012 | 0.73 |
| **Super-refractory SE (%)\*** | **25.0** | **75.0** | **0.212** | **0.01** | **Total cholesterol/HDL-C (TC/HDL-C) (AU)\*** | **5.47** | **11.3** | **0.150** | **0.03** |
| **Prolonged super-refractory SE (%)\*** | **20.7** | **79.3** | **0.138** | **0.03** | **Apolipoprotein A1/HDL-C (ApoA1/HDL-C) (AU)\*** | **3.07** | **4.78** | **0.171** | **0.03** |
| | | | | | **Esterification ratio (EC/TC) (AU)\*** | **0.64** | **0.52** | **0.159** | **0.03** |
| | | | | | **TC/Phospholipids (AU)\*** | **0.75** | **0.62** | **0.124** | **0.04** |

**Figure 2 (continued)**

| Analysis | Methods | Se | Sp | PPV | NPV | Accuracy | AUC | F1 score |
|---|---|---|---|---|---|---|---|---|
| Poor outcome at discharge | SVM classifier (11 variables) | 0.78 [0.58-0.92] | 0.75 [0.55-0.91] | 0.77 [0.64-0.92] | 0.76 [0.58-0.91] | 0.76 [0.61-0.87] | 0.75 [0.55-0.90] | 0.77 [0.61-0.92] |
| | Logistic regression | 0.69 [0.52-0.86] | 0.71 [0.45-0.92] | 0.80 [0.50-0.95] | 0.56 [0.33-0.82] | 0.69 [0.55-0.79] | 0.78 [0.67-0.88] | 0.74 [0.51-0.90] |
| | STESS (cut-off 3) | 0.49 | 0.77 | 0.71 | 0.56 | 0.62 | 0.63 | 0.58 |
| | mSTESS (cut-off 4) | 0.29 | 0.77 | 0.60 | 0.48 | 0.51 | 0.53 | 0.39 |
| Death at discharge | SVM classifier (10 variables) | 0.79 [0.50-1.0] | 0.71 [0.42-0.95] | 0.41 [0.23-0.80] | 0.94 [0.88-1.0] | 0.72 [0.52-0.91] | 0.73 [0.54-0.91] | 0.53 [0.32-0.89] |
| | STESS (cut-off 3) | 0.46 | 0.65 | 0.21 | 0.85 | 0.62 | 0.56 | 0.29 |
| | mSTESS (cut-off 4) | 0.46 | 0.78 | 0.30 | 0.88 | 0.72 | 0.62 | 0.36 |
| Recovery at 6-12 months | SVM classifier (12 variables) | 0.83 [0.60-1.0] | 0.87 [0.50-1.0] | 0.91 [0.71-1.0] | 0.83 [0.60-1.0] | 0.85 [0.67-1.0] | 0.86 [0.60-1.0] | 0.87 [0.65-1.0] |

**Figure 3**

Figure 4

**B.**

**Figure 4**

Europäisches
Patentamt

European
Patent Office

Office européen
des brevets

# EUROPEAN SEARCH REPORT

Application Number

EP 21 30 6212

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHOI JUN YOUNG ET AL: "Uric acid is a useful marker to differentiate between responsive and refractory status epilepticus", CLINICAL NEUROLOGY AND NEUROSURGERY, ELSEVIER, AMSTERDAM, NL, vol. Young184, 24 July 2019 (2019-07-24), XP085759605, ISSN: 0303-8467, DOI: 10.1016/J.CLINEURO.2019.105454 [retrieved on 2019-07-24] * abstract; supplementary Table 1 * | 1-17 | INV. G01N33/68 G16H50/30 |
| A | RATHAKRISHNAN R ET AL: "Generalised convulsive status epilepticus in Singapore: Clinical outcomes and potential prognostic markers", SEIZURE, BAILLIERE TINDALL, LONDON, GB, vol. 18, no. 3, 1 April 2009 (2009-04-01), pages 202-205, XP025997701, ISSN: 1059-1311, DOI: 10.1016/J.SEIZURE.2008.09.005 [retrieved on 2008-10-31] * abstract; Tables 4,5,6 * | 1-17 | |
| A | SATO KENICHIRO ET AL: "Status epilepticus severity score as a predictor for the length of stay at hospital for acute-phase treatment in convulsive status epilepticus", JOURNAL OF CLINICAL NEUROSCIENCE, CHURCHILL LIVINGSTONE, GB, vol. 75, 13 March 2020 (2020-03-13), pages 128-133, XP086144180, ISSN: 0967-5868, DOI: 10.1016/J.JOCN.2020.03.004 [retrieved on 2020-03-13] * p. 129, col. 2, last par.; Tables 1, 2 * | 1-17 | |

TECHNICAL FIELDS SEARCHED (IPC)

G01N
G16H

-/--

The present search report has been drawn up for all claims

| Place of search | Date of completion of the search | Examiner |
|---|---|---|
| Munich | 11 February 2022 | Hohwy, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

**EP 21 30 6212**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SCOTT MINTZER ET AL: "Conversion from enzyme-inducing antiepileptic drugs to topiramate: Effects on lipids and c-reactive protein", EPILEPSY RESEARCH, ELSEVIER SCIENCE PUBLISHERS , AMSTERDAM, NL, vol. 98, no. 1, 2 October 2011 (2011-10-02), pages 88-93, XP028340650, ISSN: 0920-1211, DOI: 10.1016/J.EPLEPSYRES.2011.10.001 [retrieved on 2011-10-05] * abstract; Table 1 * | 1-17 | |

TECHNICAL FIELDS
SEARCHED      (IPC)

The present search report has been drawn up for all claims

| Place of search | Date of completion of the search | Examiner |
|---|---|---|
| Munich | 11 February 2022 | Hohwy, Morten |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

      .....................................................................

& : member of the same patent family, corresponding
    document

EPO FORM 1503 03.82 (P04C01)

Europäisches
Patentamt

European
Patent Office

Office européen
des brevets

**Application Number**

**EP 21 30 6212**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-17(partially)**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

**1. claims: 1-17(partially)**

**Methods and devices for prognosis of the outcome of status epilepticus, comprising using at least three markers, including at least one biological marker, wherein said at least one biological marker comprises triglycerides (listed as first biological marker in claim 5).**

---

**2. claims: 1-17(partially)**

**Methods and devices for prognosis of the outcome of status epilepticus, comprising using at least three markers, including at least one biological marker, wherein said at least one biological marker comprises apolipoprotein B100 (listed as second biological marker in claim 5).**

---

**3-17. claims: 1-17(partially)**

**Methods and devices for prognosis of the outcome of status epilepticus, comprising using at least three markers, including at least one biological marker, wherein said at least one biological marker comprises the marker listed as the n'th biological marker in claim 5 for Invention n.**

---

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WILSON et al.** *Stroke,* 2005, vol. 36 (4), 777-781
- **TRINKA E ; KÄLVIÄINEN R.** 25 years of advances in the definition, classification and treatment of status epilepticus. *Seizure,* 2017, vol. 44, 65-73
- **LEITINGER M ; TRINKA E ; GIOVANNINI G et al.** Epidemiology of status epilepticus in adults: A population-based study on incidence, causes, and outcomes. *Epilepsia,* 2019, vol. 60, 53-62
- **ALKHACHROUM A ; DER-NIGOGHOSSIAN CA ; RUBINOS C ; CLAASSEN J.** Markers in Status Epilepticus Prognosis. *J Clin Neurophysiol.,* 2020, vol. 37, 422-428
- **KANTANEN A-M ; REINIKAINEN M ; PARVIAINEN I ; KÄLVIÄINEN R.** Long-term outcome of refractory status epilepticus in adults: A retrospective population-based study. *Epilepsy Res.,* 2017, vol. 133, 13-21
- **ROSSETTI AO ; LOGROSCINO G ; BROMFIELD EB.** A clinical score for prognosis of status epilepticus in adults. *Neurology,* 2006, vol. 66, 1736-1738
- **GONZALEZ-CUEVAS M ; SANTAMARINA E ; TOLEDO M et al.** A new clinical score for the prognosis of status epilepticus in adults. *Eur J Neurol.,* 2016, vol. 23, 1534-1540
- **GAO Q ; OU-YANG T ; SUN X et al.** Prediction of functional outcome in patients with convulsive status epilepticus: the END-IT score. *Crit Care,* 2016, vol. 20, 46
- **LEITINGER M ; HOLLER Y ; KALSS G et al.** Epidemiology-based mortality score in status epilepticus (EMSE). *Neurocrit Care.,* 2015, vol. 22, 273-282
- **YUAN F ; GAO Q ; JIANG W.** Prognostic scores in status epilepticus-a critical appraisal. *Epilepsia,* 2018, vol. 59 (2), 170-175
- **ROSSETTI AO ; LOGROSCINO G ; MILLIGAN TA ; MICHAELIDES C ; RUFFIEUX C ; BROMFIELD EB.** Status Epilepticus Severity Score (STESS): a tool to orient early treatment strategy. *J Neurol.,* 2008, vol. 255, 1561-1566
- **HANIN A ; LAMBRECQ V ; DENIS JA et al.** Cerebrospinal fluid and blood biomarkers of status epilepticus. *Epilepsia,* 2020, vol. 61, 6-18
- **DEGIORGIO CM ; GOTT PS ; RABINOWICZ AL ; HECK CN ; SMITH TD ; CORREALE JD.** Neuron-specific enolase, a marker of acute neuronal injury, is increased in complex partial status epilepticus. *Epilepsia,* 1996, vol. 37, 606-609
- **DEGIORGIO CM ; CORREALE JD ; GOTT PS et al.** Serum neuron-specific enolase in human status epilepticus. *Neurology,* 1995, vol. 45, 1134-1137
- **DEGIORGIO CM ; HECK CN ; RABINOWICZ AL ; GOTT PS ; SMITH T ; CORREALE J.** Serum neuron-specific enolase in the major subtypes of status epilepticus. *Neurology,* 1999, vol. 52, 746-749
- **CORREALE J ; RABINOWICZ AL ; HECK CN ; SMITH TD ; LOSKOTA WJ ; DEGIORGIO CM.** Status epilepticus increases CSF levels of neuron-specific enolase and alters the blood-brain barrier. *Neurology,* 1998, vol. 50, 1388-1391
- **FREUND Y ; BLOOM B ; BOKOBZA J et al.** Predictive value of S100-B and copeptin for outcomes following seizure: the BISTRO International Cohort Study. *PLoS ONE,* 2015, vol. 10, e0122405
- **ZHU S ; TAI C ; PETKAU TL et al.** Progranulin promotes activation of microglia/macrophage after pilocarpine-induced status epilepticus. *Brain Research,* 2013, vol. 1530, 54-65
- **SEN J ; BELLI A.** S100B in neuropathologic states: the CRP of the brain?. *J Neurosci Res.,* 2007, vol. 85, 1373-1380
- **CHALI F ; DJELTI F ; EUGENE E et al.** Inhibiting cholesterol degradation induces neuronal sclerosis and epileptic activity in mouse hippocampus. *Eur J Neurosci.,* 2015, vol. 41, 1345-1355
- **CHALI F ; MILIOR G ; MARTY S et al.** Lipid markers and related transcripts during excitotoxic neurodegeneration in kainate-treated mice. *Eur J Neurosci.,* 2019, vol. 50, 1759-1778
- **HANIN A ; BAUDIN P ; DEMERET S et al.** Disturbances of brain cholesterol metabolism: A new excitotoxic process associated with status epilepticus. *Neurobiol Dis.,* 2021, vol. 154, 105346
- **CHAUDHRY F ; HUNT RJ ; HARIHARAN P et al.** Machine Learning Applications in the Neuro ICU: A Solution to Big Data Mayhem?. *Front Neurol.,* 2020, vol. 11, 554633
- The Strengthening the Reporting of Observational Studies in Epidemiology (STROBE) Statement: Guidelines for Reporting Observational Studies. **ELM E VON ; ALTMAN DG ; EGGER M et al.** PLOS Medicine. Public Library of Science, 2007, vol. 4, e296

- **TRINKA E ; COCK H ; HESDORFFER D et al.** A definition and classification of status epilepticus--Report of the ILAE Task Force on Classification of Status Epilepticus. *Epilepsia,* 2015, vol. 56, 1515-1523
- **HIRSCH LJ ; GASPARD N ; VAN BAALEN A et al.** Proposed consensus definitions for new-onset refractory status epilepticus (NORSE), febrile infection-related epilepsy syndrome (FIRES), and related conditions. *Epilepsia,* 2018, vol. 59, 739-744
- **BRUNO A ; SHAH N ; LIN C et al.** Improving modified Rankin Scale assessment with a simplified questionnaire. *Stroke,* 2010, vol. 41, 1048-1050
- **NOBLE WS.** What is a support vector machine?. *Nature Biotechnology,* 2006, 24
- **OJALA M ; GARRIGA G.** Permutation Tests for Studying Classifier Performance. *J Mach Learn Res.,* 2010, vol. 11, 1833-1863
- **GIOVANNINI G ; MONTI G ; TONDELLI M et al.** Mortality, morbidity and refractoriness prediction in status epilepticus: Comparison of STESS and EMSE scores. *Seizure,* 2017, vol. 46, 31-37
- **SAITO T ; REHMSMEIER M.** The precision-recall plot is more informative than the ROC plot when evaluating binary classifiers on imbalanced datasets. *PLoS One.,* 2015, vol. 10, e0118432
- **MARAWAR R ; BASHA M ; MAHULIKAR A ; DESAI A ; SUCHDEV K ; SHAH A.** Updates in Refractory Status Epilepticus. *Crit Care Res Pract. 2018,* 2018, 9768949
- **CIURANS J ; GRAU-LÓPEZ L ; JIMENEZ M ; FUMANAL A ; MISIS M ; BECERRA JL.** Refractory status epilepticus: Impact of baseline comorbidity and usefulness of STESS and EMSE scoring systems in predicting mortality and functional outcome. *Seizure,* 2018, vol. 56, 98-103
- **ADIBHATLA RM ; HATCHER JF.** Altered lipid metabolism in brain injury and disorders. *Subcell Biochem.,* 2008, vol. 49, 241-268
- **MAXFIELD FR ; TABAS I.** Role of cholesterol and lipid organization in disease. *Nature,* 2005, vol. 438, 612-621
- **GASPARD N ; HIRSCH LJ ; SCULIER C et al.** New-onset refractory status epilepticus (NORSE) and febrile infection-related epilepsy syndrome (FIRES): State of the art and perspectives. *Epilepsia,* 2018, vol. 59, 745-752
- **SONNEVILLE R ; MARIOTTE E ; NEUVILLE M et al.** Early-onset status epilepticus in patients with acute encephalitis. *Medicine (Baltimore),* 2016, vol. 95, e4092
- **GASPARD N ; FOREMAN BP ; ALVAREZ V et al.** New-onset refractory status epilepticus: Etiology, clinical features, and outcome. *Neurology,* 2015, vol. 85, 1604-1613
- **PACHA MS ; ORELLANA L ; SILVA E et al.** Role of EMSE and STESS scores in the outcome evaluation of status epilepticus. *Epilepsy Behav.,* 2016, vol. 64, 140-142
- **HUTSON M.** Artificial intelligence faces reproducibility crisis. *Science,* 2018, vol. 359, 725-726
- The Lancet Respiratory Medicine. Opening the black box of machine learning. *Lancet Respir Med.,* 2018, vol. 6, 801
- **FERNÁNDEZ A ; GARCÍA S ; GALAR M ; PRATI RC ; KRAWCZYK B ; HERRERA F.** Learning from imbalanced data sets. Springer International Publishing, 2018